# EUROPEAN PATENT APPLICATION

(11) **EP 2 028 273 A1**
(43) Veröffentlichungstag der Anmeldung: **25.02.2009**
(21) Anmeldenummer: 07794002.1
(22) Anmeldetag: 21.05.2007
(51) Int. Cl.: C12N 15/11, C12Q 1/68

(54) **VERFAHREN ZUM NACHWEIS EINER INFEKTIONS DES UROGENITALBEREICHS, OLIGONUKLEOTID, OLIGONUKLEOTIDKOMBINATION, BIOCHIP UND DARAUF BASIERENDES SET**

(30) Priorität: 23.05.2006 RU 2006117378
(71) Anmelder: Closed Company "Molecular-Medicine Technologies", Moskovskaya obl. 142290 (RU)
(72) Erfinder: GRANOVSKY, Igor Eduardovich, Moskovskaya obl., 142290 (RU); BELETSKY, Igor Petrovich, Moskovskaya obl., 142290 (RU); SHLYAPNIKOVA, Elena Andreevna, Moskovskaya obl., 142290 (RU); GAVRYUSHKIN, Alexander Vladimirovich, Moskovskaya obl., 142290 (RU); BIRYUKOV, Sergey Vladimirovich, Moskovskaya obl., 142290 (RU)
(74) Vertreter: Jeck, Anton
(86) Internationale Anmeldenummer: PCT/RU2007/000248
(87) Internationale Veröffentlichungsnummer: WO 2007/136303

(57) **Zusammenfassung**

Die Erfindung bezieht sich auf ein Verfahren zur Identifizierung einer urogenitalen Infektion, das eine Probe der zu erforschenden DNS, spezifische Hin- und Rückprimer, einen biologischen Mikrochip mit Differenzieroligonukleotiden aufweist und die Durchführung von PCR in Anwesenheit der DNS und der Primer mit Sonden, die Inkubation des PCR-Produkts mit den biologischen Mikrochips unter Hybridisierungsbedingungen und die Ermittlung der urogenitalen Infektion durch die Erkennung der gebildeten Hybridisierungskomplexe auf dem Mikrochip einschließt. Die Diagnoseffektivität wird **dadurch** erhöht, dass der Mikrochip mit bestimmten Differenzieroligonukleotiden und spezifischen Primer gewählt wird, dass ein asymmetrischer PCR benutz wird, dass der Primer im Hin- und Rück-Primerpaar mit einem Marker versehen wird, dass die Erkennungsdaten erfasst werden und dass die erfassten Daten zur Identifizierung der Infektionsagenzien im urogenitalen Bereich verwendet werden.

## Beschreibung

Die Erfindung bezieht sich auf ein Verfahren nach dem Oberbegriff des Anspruchs 1 für ein Verfahren zur Identifizierung einer urogenitalen Infektion, ein Oligonukleotid, Oligonukleotidkombinationen, ein Mikrochip und ein darauf basierender Diagnosesatz zur Durchführung des Verfahrens.

### Gebiet der Technik

Die Erfindung betrifft die Mikrobiologie, die Virusforschung, die Biotechnologie, die Molekularbiologie und die Gentechnologie. Sie betrifft insbesondere die Identifizierung von Infektionspathogene (Bakterien, Viren, Pilze usw.) nach ihrer Artzugehörigkeit in biologischen Proben. Die Identifizierung der Infektionspathogene schließt eine DNS-Amplifikationsstufe und eine nachfolgende Hybridisation der amplifizierten DNS auf einem biologischen Mikrochip ein. Die Erfindung offenbart auch die Zusammensetzung der hochspezifischen DNS-Sonde und der Oligonukleotide, den Diagnosesatz und das Verfahren zur Erfassung und Auslegung der Ergebnisse.

### Stand der Technik

Heutzutage werden zur Identifizierung der Infektionspathogene je nach ihrer Artzugehörigkeit immunenzymatische Nachweismethoden (immunoenzymatic assay, IEA [1, 2]), Methoden der mikrobiologischen Analyse [3], Methoden der virologischen Analyse [4], Methoden auf Grundlage der PCR [5] eingesetzt. Dazu zählt auch die nachfolgende Längenauswertung des amplifizierten Fragments [6] oder mit nachfolgender Ermittlung der Nukleotidsequenz (Sequenzierung) [7].

Die Effektivität der IEA hängt vom Typ des zu erforschenden Materials und vom Vorhandensein der hochspezifischen monoklonalen Antikörper für jede Pathogenart ab. Wegen der nahen Antigenverwandtschaft von unterschiedlichen Pathogenen sind die Forschungsergebnisse der Infektionsagenzien nicht immer zuverlässig.

Die Methoden der mikrobiologischen und der virologischen Analyse sind Kulturalmethoden und setzen dementsprechend den Einsatz von Sonderausrüstungen und hochqualifizierten Fachkräften voraus. Die Analyse nimmt einige Tage in Anspruch und hängt mit den besonderen Sicherheitsmaßnahmen wegen der erhöhten biologischen Gefahr zusammen.

Der wesentliche Mangel der konventionellen PCR-Methode [6] ist der visuelle Charakter bei der Einschätzung der Ergebnisse. Das kann ihre falsche Auslegung verursachen. Wenn die Nukleotidsequenz nach diesen Methoden ermittelt wird, so hängen die Analyseprozeduren mit großen Zeit- und Geldaufwendungen zusammen. Außerdem setzen sie ebenfalls den Einsatz von Sonderausrüstungen und hochqualifizierten Fachkräften voraus.

Es ist die Erfindung der Diagnose von urogenitalen Krankheiten bekannt, bei der in einer Probe die Komplexe erforscht werden, welche grampositive Bakterien und andere Mikroorganismen, z. B. Hefe, Protozoen, Mykoplasma und gramnegative Bakterien aufweisen [8].

Es ist eine Erfindung bekannt, welche ein Diagnoseverfahren offenbart, bei dem die Nukleinsäuregruppen zur Bildung von Proben benutzt werden. Die Proben werden mit dem Abschnitt 16S rRNS oder 16S rDNS der Ureaplasma urealyticum [9] hybridisiert.

Es ist eine Erfindung bekannt, bei der gleichzeitig eine Menge von Mikroorganismen erkannt werden. Die Mikroorganismen werden aus der Gruppe Neisseria gonorrhoeae, Neisseria meningitidis, Haemophilus ducreyi, Branhamella catarrhalis, Bordetella pertussis, Haemophilus influenzae, Streptococcus pneumoniae, Streptococcus agalactiae, Campylobacter jejuni, Campylobacter coli oder ihrer Kombinationen gewählt. Gemäß dieser Erfindung werden die Nukleinsäuren vieler Mikroorganismen in Kontakt mit einer Membran gebracht. Die Membran enthält solche Abschnitte, auf denen Nukleinsäureproben aufgetragen werden. Die Nukleinsäuren wurden dem Transkriptionsabschnitt im Gen zwischen der 16S und 23S rRNS vom Gen des prokaryotischen Mikroorganismus entnommen, welcher 15 bis 100 Nukleotide [10] enthielten.

In einer Erfindung [11] sind die Primer zur Amplifikation der Abschnitte 16S - 23S rRNS und die nachfolgende Hybridisation zur Erkennung von Mikroorganismen offenbart, die der Stammgruppe Mycobacterium, Chlamydia, Listeria, Brucella und Yersinia enterolitica angehören.

In einer Erfindung [12] ist die technische Lösung im Zusammenhang mit der Echtzeit-Anwendung von PCR zur Diagnose von beliebigen Eubakterien offenbart. Dabei wird der hochkonservative Abschnitt des 16S rRNS Gens benutzt.

In einer Erfindung [13] sind Oligonukleotide und Amplifikationsprimer zur Erkennung des Vorhandenseins der Bakterien oder Pilze offenbart, welcher der Gruppe Enterococcus faecium, Listeria monocytogenes, Neissera meningitidis, Streptococcus agalactiae, Candida albicans, Enterococcus genus, Neisseria genus, Staphylococcus genus, Streptococcus genus und Candida genus angehören. Die Amplifikation erfolgt mittels PCR und anderer Systeme: LCR, NASBA, 3SR, SDA, bDNA, (TMA), CPT, geschachtelte PCR und Multiplex-PCR.

Es sind Primer und PCR-Sätze zur Identifikation von Herpes simplex virus Typ 1 und 2 [23, 24] sowie Herpes simplex virus Typ 4 sowie zur Identifikation von Epstein-Barr-Virus [25] bekannt. Der wesentliche Teil der bekannten technischen Lösungen betrifft die Lösung von spezifischen Aufgaben, die mit der Suche entweder nach neuen Primerarten oder nach den Verfahrenstechniken ihrer Anwendung auf dem Gebiet der Diagnose zusammenhängen [26 - 37].

Die der angemeldeten Erfindung am nächsten liegende Lösung bietet eine Erfindung [14]. In dieser Erfindung wird das Verfahren zur Positionsermittlung von einer Menge der Proben mit unterschiedlichen Genotypen auf der Oberfläche von einem Mikrochip beschrieben. Diese Methode betrifft die Anwendung von der Multiprimer-Polymerase-Kettenreaktion (PCR) unter Einsatz der fluoreszent markierten Primer, um die amplifizierten Einketten-DNS zu bekommen. Unter der Multiprimer-PCR wird normalerweise der Vorgang der gemeinsamen Amplifikation mehrerer DNS-Matrizen in einem Reaktionsmedium unter Einsatz von einigen Primerpaaren verstanden. Dies ermöglicht es, die Selektion (Screening) auf einmal nach mehreren Infektionserregern vorzunehmen.

Jedoch ist dieses Verfahren ohne wesentliche Nacharbeiten unanwendbar. Die Nacharbeiten sind erforderlich, um einen verhältnismäßig günstigen Mikrochip oder einen Multichip zu erzeugen. Der Multichip besteht dabei aus einigen Chips. Dies ist zur Identifikation der urogenitalen Infektionen erforderlich. Die Grundsätze der Durchführung von Multiprimer-PCR weisen spezifische Besonderheiten auf. Sie hängen mit der Temperaturauswahl zur Durchführung von PCR, mit der PrimerLänge und mit der Wahl der Primer-Reihenfolgen zusammen. Diese Faktoren üben keinen Einfluss während der Amplifikation aufeinander aus. Außerdem wird die Effektivität der Diagnose in vieler Hinsicht durch die Auswahl der Pathogenorganismen festgelegt. Anhand dieser Pathogenorganismen wird die Identifikation der gemischten urogenitalen Infektionen vorgenommen.

Es ist Aufgabe der Erfindung, ein Verfahren zur Ermittlung der urogenitalen Mischinfektion anhand der Identifikation von bis zu 15 Pathogenarten an biologischen Mikrochips zu schaffen, das die Diagnoseeffektivität erhöht. Daher soll ein biologischer Mikrochip und Multichip geschaffen werden, der eine Durchführung der Diagnose unter Laborbedingungen ermöglicht, ohne komplizierte Diagnoseausrüstungen anwenden zu müssen.

Die gestellte Aufgabe wird beim Verfahren durch die Merkmale des Anspruchs 1 gelöst.

Das Verfahren zur Identifizierung der urogenitalen Infektion umfasst folgende Schritte:
- die Sicherstellung
   - der Probe der zu erforschenden DNS;
   - der Hin- und Rückprimer, welche für die Identifikation der Infektionsagenzien im urogenitalen Bereich spezifisch sind, und
   - des biologischen Mikrochips, welcher die Differenzieroligonukleotide aufweist,
- die Durchführung von PCR in Anwesenheit der DNS-Probe und der Primer mit Sonden, die für die Identifikation der Infektionsagenzien spezifisch sind,
- die Inkubation des PCR-Produkts mit dem biologischen Mikrochip unter den Hybridisierungsbedingungen und
- die Ermittlung der urogenitalen Infektion durch die Erkennung der gebildeten Hybridisierungskomplexe auf dem Mikrochip.

Dabei enthält der biologische Mikrochip die Differenzieroligonukleotide mit den Sequenzen SEQ ID NO:59-87. Als spezifische Primer werden solche Primer verwendet, deren Sequenz aus der Gruppe gewählt wird, die aus SEQ ID NO: 1-58 besteht. Als PCR wird ein asymmetrischer PCR benutzt. Dabei enthält ein Primer im Hin- und Rück-Primerpaar einen Marker. Dieser Marker wird nach der DNS-Hybridisierung erkannt. Die Erkennungsdaten werden erfasst. Die Erfassungsdaten werden zur Identifikation der Infektionsagenzien im urogenitalen Bereich verwendet.

Ein anderer Gegenstand der Erfindung ist ein spezifisches Oligonukleotid als PCR-Primer zur Identifizierung des Infektionsagens je nach der Artzugehörigkeit im Verfahren zur Identifizierung der urogenitalen Infektion mit den Sequenzen von SEQ ID NO: 1 bis SEQ ID NO: 58.

Ein weiterer Gegenstand der Erfindung ist ein Differenzier-Oligonukleotid als eine Sonde zur Identifizierung des Infektionsagens je nach der Artzugehörigkeit im Verfahren zur Identifizierung der urogenitalen Infektion mit den Sequenzen von SEQ ID NO: 59 bis SEQ ID NO: 87.

Ein weiterer Gegenstand der Erfindung ist eine Oligonukleotidkombination zur Identifizierung des Infektionsagens Chlamydia trachomatis je nach der Artzugehörigkeit.

Diese Kombination umfasst Oligonukleotide mit den Sequenzen entweder von SEQ ID NO: 59 und SEQ ID NO: 1 und SEQ ID NO: 2, oder SEQ ID NO: 60 und SEQ ID NO: 3 und SEQ ID NO: 4.
Ein weiterer Gegenstand der Erfindung ist eine Oligonukleotidkombination zur Identifizierung des Infektionsagens Neisseria gonorrhoeae je nach der Artzugehörigkeit. Die Oligonukleotidkombination umfasst Oligonukleotide mit den Sequenzen von entweder SEQ ID NO: 61 und SEQ ID NO: 5 und SEQ ID NO: 6, oder SEQ ID NO: 62 und SEQ ID NO: 7 und SEQ ID NO: 84, oder SEQ ID NO: 63 und SEQ ID NO: 9 und SEQ ID NO: 10.

Ein weiterer Gegenstand der Erfindung ist eine Oligonukleotidkombination zur Identifizierung des Infektionsagens Trichomonas vaginalis je nach der Artzugehörigkeit. Die Oligonukleotidkombination umfasst Oligonukleotide mit den Sequenzen von entweder SEQ ID NO: 64 und SEQ ID NO: 11 und SEQ ID NO: 12, oder SEQ ID NO: 65 und SEQ ID NO: 13 und SEQ ID NO:14.
Ein weiterer Gegenstand der Erfindung ist eine Oligonukleotidkombination zur Identifizierung des Infektionsagens Ureaplasma urealyticum und/oder Ureaplasma parvum je nach der Artzugehörigkeit. Die Oligonukleotidkombination umfasst Oligonukleotide mit den Sequenzen von entweder SEQ ID NO: 66 und SEQ ID NO: 15 und SEQ ID NO: 16, oder SEQ ID NO: 67 und SEQ ID NO: 17 und SEQ ID NO: 18.

Ein weiterer Gegenstand der Erfindung ist eine Oligonukleotidkombination zur Identifizierung des Infektionsagens Candida albicans je nach der Artzugehörigkeit. Diese Oligonukleotidkombination umfasst Oligonukleotide mit den Sequenzen von entweder SEQ ID NO: 68 und SEQ ID NO: 19 und SEQ ID NO: 20, oder SEQ ID NO: 69 und SEQ ID NO: 21 und SEQ ID NO: 22.

Ein weiterer Gegenstand der Erfindung ist eine Oligonukleotidkombination zur Identifizierung des Infektionsagens Mycoplasma genitalium je nach der Artzugehörigkeit. Diese Oligonukleotidkombination umfasst Oligonukleotide mit den Sequenzen von entweder SEQ ID NO: 70 und SEQ ID NO: 23 und SEQ ID NO: 24, oder SEQ ID NO: 71 und SEQ ID NO: 25 und SEQ ID NO: 26, oder SEQ ID NO: 72 und SEQ ID NO: 27 und SEQ ID NO: 28.

Ein weiterer Gegenstand der Erfindung ist eine Oligonukleotidkombination zur Identifizierung des Infektionsagens Mycoplasma hominis je nach der Artzugehörigkeit. Diese Oligonukleotidkombination umfasst Oligonukleotide mit den Sequenzen von entweder SEQ ID NO: 73 und SEQ ID NO: 29 und SEQ ID NO: 30, oder SEQ ID NO: 74 und SEQ ID NO: 31 und SEQ ID NO: 32, oder SEQ ID NO: 75 und SEQ ID NO: 33 und SEQ ID NO: 34.

Ein weiterer Gegenstand der Erfindung ist eine Oligonukleotidkombination zur Identifizierung des Infektionsagens Gardnerella vaginalis je nach der Artzugehörigkeit. Diese Oligonukleotidkombination umfasst Oligonukleotide mit den Sequenzen von entweder SEQ ID NO: 76 und SEQ ID NO: 35 und SEQ ID NO: 36, oder SEQ ID NO: 77 und SEQ ID NO: 37 und SEQ ID NO: 38, oder SEQ ID NO: 78 und SEQ ID NO: 39 und SEQ ID NO: 40.
Ein weiterer Gegenstand der Erfindung ist eine Oligonukleotidkombination zur Identifizierung des Infektionsagens Streptococcus agalactiae je nach der Artzugehörigkeit. Die Oligonukleotidkombination umfasst Oligonukleotide mit den Sequenzen von SEQ ID NO: 79 und SEQ ID NO:.41 und SEQ ID NO: 42.

Ein weiterer Gegenstand der Erfindung ist eine Oligonukleotidkombination zur Identifizierung des Infektionsagens Streptococcus agalactiae und/oder Streptococcus pyogenes je nach der Artzugehörigkeit. Diese Oligonukleotidkombination umfasst Oligonukleotide mit den Sequenzen von SEQ ID NO: 80 und SEQ ID NO: 43 und SEQ ID NO: 44.

Ein weiterer Gegenstand der Erfindung ist eine Oligonukleotidkombination zur Identifizierung des Infektionsagens Human Herpesvirus 1 und/oder Human Herpesvirus 2 je nach der Artzugehörigkeit. Die Oligonukleotidkombination umfasst Oligonukleotide mit den Sequenzen von entweder SEQ ID NO: 81 und SEQ ID NO: 45 und SEQ ID NO: 46, oder SEQ ID NO: 82 und SEQ ID NO: 47 und SEQ ID NO: 48.

Ein weiterer Gegenstand der Erfindung ist eine Oligonukleotidkombination zur Identifizierung des Infektionsagens Human Herpesvirus 4 je nach der Artzugehörigkeit. Die Oligonukleotidkombination umfasst Oligonukleotide mit den Sequenzen von entweder SEQ ID NO: 83 und SEQ ID NO: 49 und SEQ ID NO: 50, oder SEQ ID NO: 84 und SEQ ID NO: 51 und SEQ ID NO: 52, oder SEQ ID NO: 85 und SEQ ID NO: 53 und SEQ ID NO: 54.

Ein weiterer Gegenstand der Erfindung ist eine Oligonukleotidkombination zur Identifizierung des Infektionsagens Human Herpesvirus 5 je nach der Artzugehörigkeit. Die Oligonukleotidkombination umfasst Oligonukleotide mit den Sequenzen von entweder SEQ ID NO: 86 und SEQ ID NO: 55 und SEQ ID NO: 56, oder SEQ ID NO: 87 und SEQ ID NO: 57 und SEQ ID NO: 58.

Ein weiterer Gegenstand der Erfindung ist ein biologischer Mikrochip zur Identifizierung der Infektionsagenzien je nach ihrer Artzugehörigkeit. Der biologische Mikrochip stellt einen Träger dar, auf dem die Differenzieroligonukleotide mit einer oder mehr Sequenzen von SEQ ID NO: 59 bis SEQ ID NO: 87 immobil gemacht sind.

Ein weiterer Gegenstand der Erfindung ist ein Satz zur Identifizierung der Infektionsagenzien je nach ihrer Artzugehörigkeit. Der Satz enthält:
a) mindestens einen individuellen biologischen Mikrochip zur Durchführung einer einmaligen Diagnose oder einen biologischen Multichip zur Durchführung von Parallel- oder Serienselektion (Screening). Der Chip enthält Differenzieroligonukleotide mit einer oder mehr Sequenzen von SEQ ID NO: 59 bis SEQ ID NO: 87.
b) Reagenzien, welche aus den Reagenzien zur Probevorbereitung, Reagenzien zur PCR-Durchführung, Reagenzien zur Durchführung der Hybridisierung sowie ihre Kombinationen gewählt sind und
c) spezifische Oligonukleotide als PCR-Primer zur Identifizierung des Infektionsagens je nach der Artzugehörigkeit mit den Sequenzen, die aus dem Bereich von SEQ ID NO: 1 bis SEQ ID NO: 58 gewählt sind.

Ein weiterer Gegenstand der Erfindung ist ein Satz, der, außer dem biologischen Mikrochip oder Multichip, Reagenzien zur Probevorbereitung, Reagenzien zur PCR-Durchführung, Reagenzien zur Durchführung der Hybridisierung sowie ihren Kombinationen und spezifische Oligonukleotiden als PCR-Primer zur Identifizierung des Infektionsagens je nach der Artzugehörigkeit, zusätzlich eine Einrichtung zur Durchführung der Hybridisierung und/oder eine Einrichtung zum Scannen und zur Auslegung der Analyseergebnisse enthält. Dabei wird zum Scannen eine Einrichtung verwendet, welche einer Gruppe von Vorrichtungen angehört. Die Gruppe beinhaltet auch einen Handscanner, einen Scanner für individuelle Chips, einen Flachbettscanner für Multichips, einen Scanner für Scheiben-Multichips und einen PC als Mittel zur Auslegung und Speicherung der Diagnoseergebnisse.

Die Erfindung wird anhand von in den Zeichnungen dargestellten Ausführungsbeispielen näher erläutert. Es zeigen:

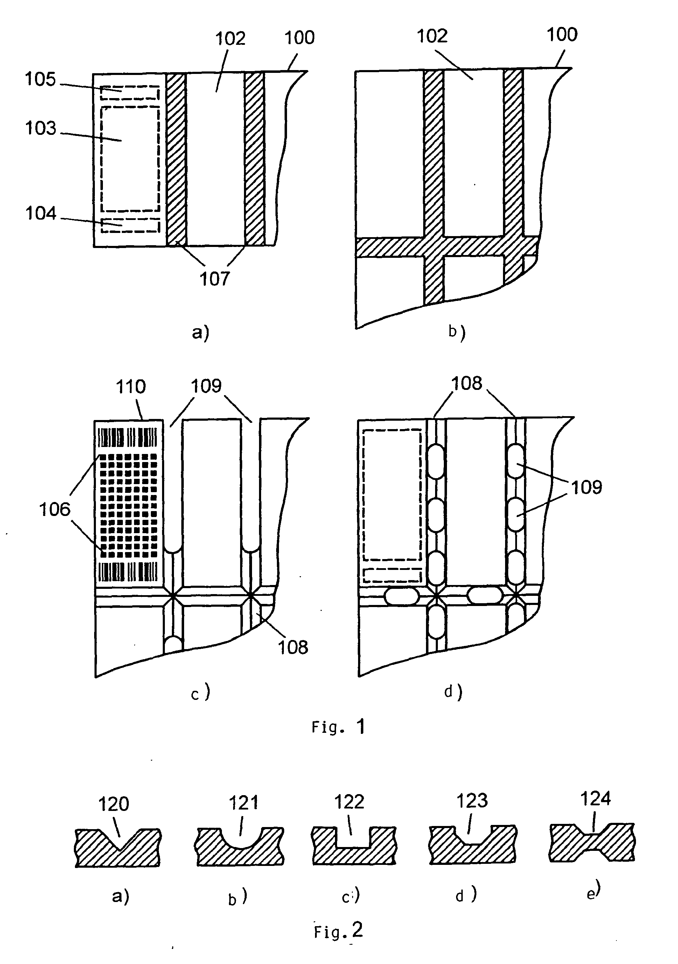

- Fig.1: eine Anordnung der Grundelemente des Multichips auf der Trägeroberfläche:
a) und b) Zonenverteilung auf dem Träger eines Multichips,
c) eine Ausführung des Multichips mit einer Kombination von Aussparungen und durchgehenden Bohrungen zwischen den Chips sowie mit der Anordnung von zwei Identifizierungsmerkmalen und
d) eine Ausführung des Multichips mit Aussparungen zwischen den Chips sowie mit der Anordnung von einem Identifizierungsmerkmal,
- Fig. 2: einen Multichipträger im Schnitt für verschiedene Ausführungen der Aussparungen:
a) dreieckige Aussparung,
b) Kugelaussparung,
c) rechteckige Aussparung,
d) Polygonaussparung und
e) Ausführung mit der Aussparung an beiden Seiten des Trägers,
- Fig. 3: eine Anordnung der Grundelemente des Multichips auf der Trägeroberfläche, wobei die gemeinsame Zone auf dem Träger gebildet ist:
a) Zonenverteilung auf dem Träger des Multichips,
b) eine Ausführung des Multichip mit einer Kombination von Aussparungen und durchgehenden Bohrungen zwischen den Chips sowie mit der Anordnung von drei Identifizierungsmerkmalen und
c) eine Ausführung des Multichips mit einer Kombination von Aussparungen und durchgehenden Bohrungen zwischen den Chips sowie mit der Anordnung von zwei Identifizierungsmerkmalen.

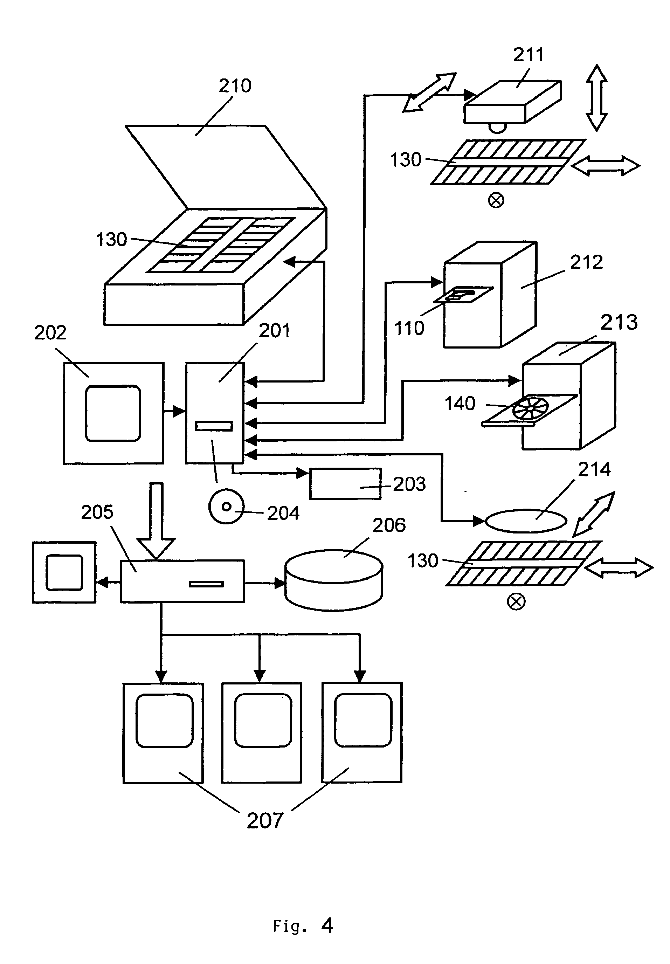

- Fig. 4: einen Übersichtsschaltplan des Systems zur Sammlung, Bearbeitung und Übertragung der von Multichips abgelesenen Daten auf einen individuellen PCs der Benutzer.

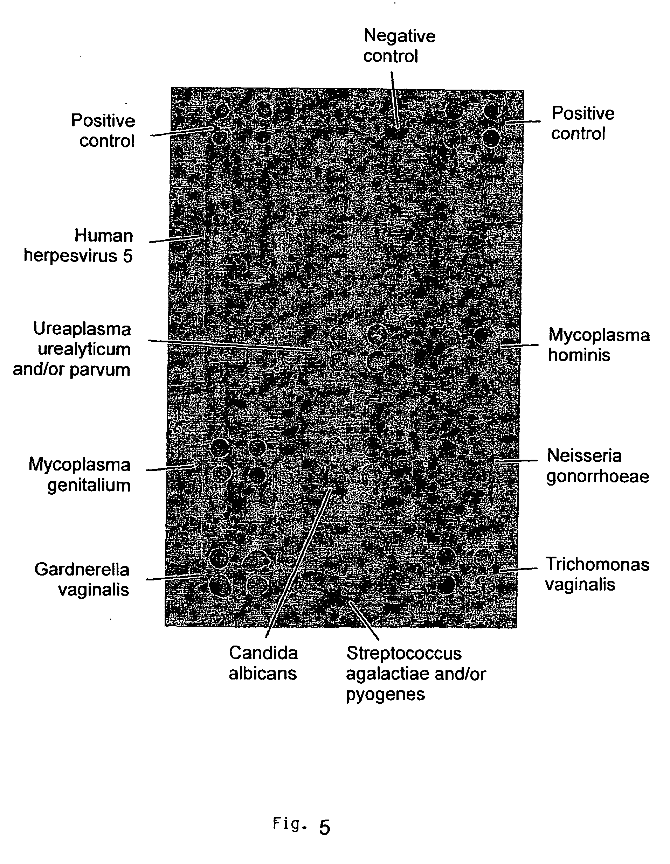

- Fig. 5: eine Abbildung der Punkte bei der Erkennung einer Versuchsfolie (experimental slide) zur Prüfung der Wirksamkeit der Wahl von Sonden und Primer sowie für positive und negative Kontrollmittel zur Diagnose der urogenitalen Infektion und

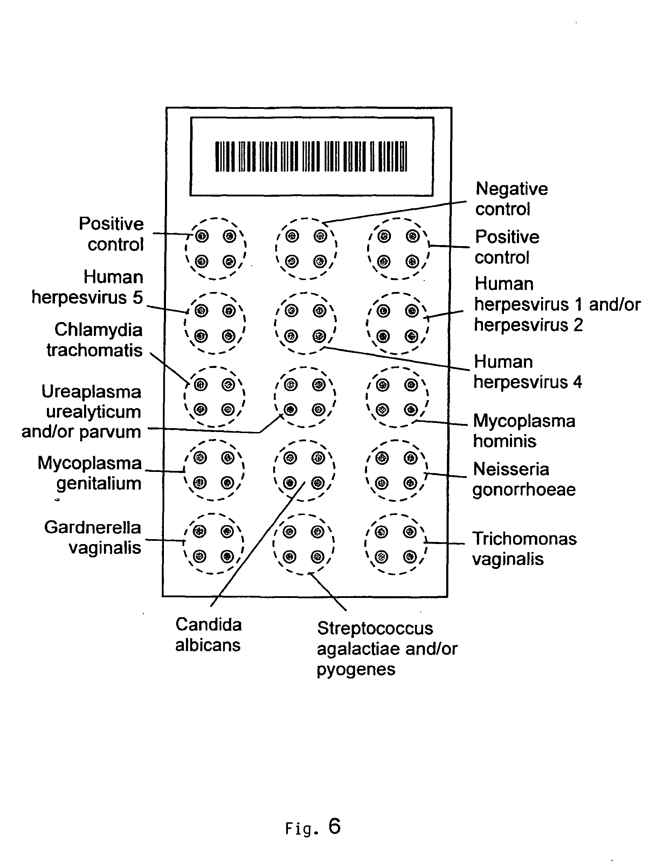

- Fig. 6: ein Strukturschaubild des medizinischen Chips.

### Abkürzungen

### IEA - immunenzymatische Analyse

Positionen 100 betreffen Konstruktionselemente des Multichips.
Positionen 200 betreffen das System zur Analyse der Diagnoseergebnisse.

### Beschreibung der Erfindung

Im Allgemeinen schließt das Verfahren zur Identifizierung der Infektionsagenzien je nach ihrer Artzugehörigkeit vorwiegend im urogenitalen Bereich folgende Schritte ein:
- Targetauswahl,
- Primer- und Sondenauswahl,
- Sonden- und Primersynthese,
- Aufbereitung des biologischen Mikrochips (z. B. Oberflächenmodifizierung),
- Auftragen der Sonden,
- DNS-Aussonderung,
- DNS-Amplifikation anhand von PCR,
- DNS-Hybridisierung auf dem Mikrochip,
- Erkennung und Erfassung der Ergebnisse,
- Identifikation und Auslegung der Ergebnisse.

### Targetauswahl

Beim Studium der Diagnoseverfahren der urogenitalen Infektionen und der bekannten Diagnosemikrochips wurde festgestellt, dass die meisten bekannten Verfahren, die auf der Wahl von einer Liste der Pathogenorganismen zur Diagnose beruhen, die Tatsache nicht berücksichtigen, dass in der letzten Zeit kräftiges Wachstum der mit der urogenitalen Mischinfektion zusammenhängenden Erkrankungen beobachtet wird [38].

Die nichtoffensichtliche Lösung im Rahmen dieser Erfindung umfasst die Wahl der Liste von Pathogenorganismen. Anhand dieser Pathogenorganismen wird die Identifikation der urogenitalen Mischinfektion vorgenommen. Die Liste aus der Tabelle Nr. 1 wurde so erstellt, dass sie die Mängel der bekannten Erfindungen beseitigt. Neben den Bakterien, Hefen und Pilzen, denen man in der urogenitalen Diagnose begegnet, enthält diese Liste die Viren, Human Herpesvirus 1 und/oder Human Herpesvirus 2, Human Herpesvirus 4 und Human Herpesvirus 5. Diese Gesamtheit von Viren, Bakterien, Hefen und Pilzen ermöglicht es, die technische Lösung der Erfindung zu realisieren, welche gleichzeitig die Erhöhung der Diagnoseeffektivität sicherstellt. Sie ermöglicht es, gleichzeitig, schnell und mit Minimalaufwand von 1 bis zu 15 Pathogenen zu diagnostizieren. Die Analyse beruht auf der Identifikation von bis zu 26 DNS-Abschnitte, welche für diese Pathogenorganismen spezifisch sind.

**Tabelle 1. Liste der Pathogenorganismen und Targets.**

| Pos. | Organismus | Gen | Abkürzung |
|---|---|---|---|
| 1 | Chlamydia trachomatis | Gen des Inklusionsmembranproteins A | incA |
| 2 | Chlamydia trachomatis | Gen des Inklusionsmembranproteins F | incF |
| 3 | Neisseria gonorrhoeae | Gen der Deoxyzytosinemethylase | dcmG |
| 4 | Neisseria gonorrhoeae | Pseudogen, äußeres Membranprotein Klasse 1 | porA |
| 5 | Neisseria gonorrhoeae | Gene der 16S und 23S ribosomaler RNS | 16S-23S |
| 6 | Trichomonas vaginalis | Interner transkribierbarer Spacer der Gene 5.8S und 18S rRNS | ITS1 |
| 7 | Trichomonas vaginalis | Wiederholter DNS-Abschnitt | Wiederholter DNS-Abschnitt |
| 8 | Ureaplasma urealyticum\parvum | Gen der Komponente vom Ureasekomplex UreB | ureB |
| 9 | Ureaplasma urealyticum\ parvum | Gen des Elongationsfaktors Tu | tuf |
| 10 | Candida albicans | Interner transkribierbarer Spacer der Gene 5.8S und 18S rRNS | ITS1 |
| 11 | Candida albicans | Interner transkribierbarer Spacer der Gene 5.8S und 23S rRNS | ITS2 |
| 12 | Mycoplasma genitalium | Spacer zwischen den Genen 16S und 23S rRNS | 16S-23S ITS |
| 13 | Mycoplasma genitalium | Gen Glyzeraldehyde-3-Phosphat Dehydrogenase | gap |
| 14 | Mycoplasma genitalium | Lipoproteingen | Ipp |
| 15 | Mycoplasma hominis | Spacer zwischen den Genen 16S und 23S rRNS | 16S-23S ITS |
| 16 | Mycoplasma hominis | Gen von Glyzeraldehyde-3-Phosphat Dehydrogenase | gap |
| 17 | Mycoplasma hominis | Gen vom Rezeptor von der signalerkennenden Partikel | ftsY |
| 18 | Gardnerella vaginalis | Spacer zwischen den Genen 16S und 23S rRNS | 16S-23S ITS |
| 19 | Gardnerella vaginalis | Gen von Hitzeschock-Protein 60 | hsp60 |
| 20 | Streptococcus agalactiae | CAMP-Faktor-Gen | cfb |
| 21 | Streptococcus agalactiae\ pyogenes | Gen des Peptidase C5A-Präkusors | scpB, scpA |
| 22 | Human Herpesvirus 1/2 | DNS-Polymerase-Gen | UL30 |
| 23 | Human Herpesvirus 4 | Proteingen EBNA-1 (Nuklearantigen des Epstein-Barr-Virus) | EBNA-1 |
| 24 | Human Herpesvirus 4 | Replikationsorigin 1 | oriP |
| 25 | Human Herpesvirus 5 | Früh-Transkriptionsregler (Immediately-early (pre-early) transcription regulator) | UL123 |
| 26 | Human Herpesvirus 5 | Gen 150 | UL150 |

Die Wahl von spezifischen und Differenzier-Oligonukleotiden und ihre mögliche Kombinationen.

### Die Wahl von spezifischen Oligonukleotiden für PCR

Die Primer wurden unter Berücksichtigung von hochspezifischen Anforderungen an die konservativen Genabschnitte der zu erforschenden Pathogene gewählt. Das ermöglicht es, den DNS-Abschnitt des Pathogenorganismus selektiv und direkt aus der
ausgesonderten biologischen Probe zu amplifizieren. Dabei wurde aus den an die Primer standardmäßig gestellten Anforderungen ausgegangen. Das sind unter anderem folgende Anforderungen: Das Fehlen von hochstabilen Sekundärstrukturen, geringer Unterschied in den Schmelztemperaturen von max. 2 - 3 °C sowie die Wahl der Primerlänge im Bereich von 15 bis 55 BP.

Um die Primer zusammenzustellen, wurden Sequenzen gewählt, welche für den identifizierbaren Pathogenorganismus kennzeichnend sind und in der Datenbank der Nukleotidsequenzen der GenBank (http://ncbi.nlm.nih.gov) aufgeführt sind. Hier wurden die am meisten konservative Abschnitte gewählt, die früher nicht benutzt wurden und für die Zusammenstellung von spezifischen und Differenzier-Oligonukleotiden zur Erzeugung von biologischen Chips nichtoffensichtlich sind.

Jedes Oligonukleotidepaar für PCR, spezifisch für ein bestimmtes Pathogentarget, besteht aus einem unmarkierten spezifischen Oligonukleotid und einem markierten spezifischen Oligonukleotid. Das unmarkierte spezifische Oligonukleotid enthält keinen Marker, der für die nachfolgende Analyse und Ergebnisdeutung erforderlich ist. Dieses spezifische Oligonukleotid gehört der Liste von Sequenzen mit einer beliebigen ungeraden Nummer mit SEQ ID NO: 1 bis SEQ ID NO: 57 an. Das markierte spezifische Oligonukleotid enthält eine bzw. mehrere Marker, die für die nachfolgende Analyse und Ergebnisdeutung erforderlich sind. Dieses spezifische Oligonukleotid gehört der Liste von Sequenzen mit einer beliebigen geraden Nummer von SEQ ID NO: 2 bis SEQ ID NO: 58 an. Es folgt sofort nach dem ersten spezifischen Oligonukleotid im Paar.

### Die Wahl von Differenzier-Otigonukteotiden

Die Differenzieroligonukleotide wurden unter Berücksichtigung von hochspezifischen Anforderungen an die konservativen Genabschnitte der zu erforschenden Pathogene gewählt. Dies ermöglicht es, die selektive Hybridisierung der DNS-Abschnitte vorzunehmen. Die DNS-Abschnitte werden mit spezifischen Oligonukleotiden anhand von PCR unmittelbar aus biologischen Proben amplifiziert. Die Wahl erfolgte auch aufgrund der fehlenden hochstabilen Sekundärstrukturen. Dabei betrug die Länge der Oligonukleotide 15 bis 55 BP.

Die Differenzieroligonukleotide sind durch ihre Artspezifität gegenüber den Pathogenen vorwiegend im urogenitalen Bereich gekennzeichnet.

Es ist zulässig, die Oligonukleotide zu benutzen, die gegenüber den in der Sequenzenliste angegebenen Oligonukleotiden mindestens zu 80 %, vorzugsweise jedoch mindestens zu 90 % und am besten mindestens zu 95 % homolog sind. Es dürfen auch Oligonukleotide eingesetzt werden, die Deletionen bzw. Einfügung von einem bzw. mehreren Nukleotiden aufweisen.

### Kombinationen der Oligonukleotide

1. Zur Identifizierung der Infektionsagenzien je nach ihrer Artzugehörigkeit, vorwiegend im urogenitalen Bereich, und zur spezifischen Erkennung von Bakterien Chlamydia trachomatis ist eine beliebige von zwei Kombinationen verwendet. Diese Kombination schließt ein Differenzier-Oligonukleotid als Sonde und zwei spezifische Oligonukleotide als PCR-Primer ein. Eine dieser Kombinationen enthält ein Differenzier-Oligonukleotid SEQ ID NO: 59 und zwei spezifische Oligonukleotide SEQ ID NO: 1 und SEQ ID NO: 2. Die andere Kombination enthält das Differenzier-Oligonukleotid SEQ ID NO: 60 und zwei spezifische Oligonukleotide SEQ ID NO: 3 und SEQ ID NO: 4.
2. Zur Identifizierung der Infektionsagenzien je nach ihrer Artzugehörigkeit, vorwiegend im urogenitalen Bereich, und zur spezifischen Erkennung von Bakterien Neisseria gonorrhoeae ist eine beliebige von zwei Kombinationen verwendet. Diese Kombination schließt ein Differenzier-Oligonukleotid als Sonde und zwei spezifische Oligonukleotide als PCR-Primer ein. Eine dieser Kombinationen enthält ein Differenzier-Oligonukleotid SEQ ID NO: 61 und zwei spezifische Oligonukleotide SEQ ID NO: 5 und SEQ ID NO: 6. Die andere Kombination enthält das Differenzier-Oligonukleotid SEQ ID NO: 62 und zwei spezifische Oligonukleotide SEQ ID NO: 7 und SEQ ID NO: 84. Die dritte Kombination umfasst das Differenzier-Oligonukleotid SEQ ID NO: 63 und zwei spezifische Oligonukleotide SEQ ID NO: 9 und SEQ ID NO: 10.
3. Zur Identifizierung der Infektionsagenzien je nach ihrer Artzugehörigkeit, vorwiegend im urogenitalen Bereich, und zur spezifischen Erkennung von Bakterien Trichomonas vaginalis ist eine beliebige von zwei Kombinationen verwendet. Diese Kombination schließt ein Differenzier-Oligonukleotid als Sonde und zwei spezifische Oligonukleotide als PCR-Primer ein. Eine dieser Kombinationen enthält ein Differenzier-Oligonukleotid SEQ ID NO: 64 und zwei spezifische Oligonukleotide SEQ ID NO: 11 und SEQ ID NO: 12. Die andere Kombination enthält das Differenzier-Oligonukleotid SEQ ID NO: 65 und zwei spezifische Oligonukleotide SEQ ID NO: 13 und SEQ ID NO: 14.
4. Zur Identifizierung der Infektionsagenzien je nach ihrer Artzugehörigkeit, vorwiegend im urogenitalen Bereich, und zur spezifischen Erkennung von Bakterien Ureaplasma urealyticum und/oder Ureaplasma parvum ist eine beliebige von zwei Kombinationen verwendet. Diese Kombination schließt ein Differenzier-Oligonukleotid als Sonde und zwei spezifische Oligonukleotide als PCR-Primer ein. Eine dieser Kombinationen enthält ein Differenzier-Oligonukleotid SEQ ID NO: 66 und zwei spezifische Oligonukleotide SEQ ID NO: 15 und SEQ ID NO: 16. Die andere Kombination enthält das Differenzier-Oligonukleotid SEQ ID NO: 67 und zwei spezifische Oligonukleotide SEQ ID NO: 17 und SEQ ID NO: 18.
5. Zur Identifizierung der Infektionsagenzien je nach ihrer Artzugehörigkeit, vorwiegend im urogenitalen Bereich, und zur spezifischen Erkennung von Pilzen Candida albicans ist eine beliebige von zwei Kombinationen verwendet. Diese Kombination schließt ein Differenzier-Oligonukleotid als Sonde und zwei spezifische Oligonukleotide als PCR-Primer ein. Eine dieser Kombinationen enthält ein Differenzier-Oligonukleotid SEQ ID NO: 68 und zwei spezifische Oligonukleotide SEQ ID NO: 19 und SEQ ID NO: 20. Die andere Kombination enthält das Differenzier-Oligonukleotid SEQ ID NO: 69 und zwei spezifische Oligonukleotide SEQ ID NO: 21 und SEQ ID NO: 22.
6. Zur Identifizierung der Infektionsagenzien je nach ihrer Artzugehörigkeit, vorwiegend im urogenitalen Bereich, und zur spezifischen Erkennung von Bakterien Mycoplasma genitalium ist eine beliebige von zwei Kombinationen verwendet. Diese Kombination schließt ein Differenzier-Oligonukleotid als Sonde und zwei spezifische Oligonukleotide als PCR-Primer ein. Eine dieser Kombinationen enthält das Differenzier-Oligonukleotid SEQ ID NO: 70 und zwei spezifische Oligonukleotide SEQ ID NO: 23 und SEQ ID NO: 24. Die andere Kombination enthält das Differenzier-Oligonukleotid SEQ ID NO: 71 und zwei spezifische Oligonukleotide SEQ ID NO: 25 und SEQ ID NO: 26. Die dritte Kombination umfasst das Differenzier-Oligonukleotid SEQ ID NO: 72 und zwei spezifische Oligonukleotide SEQ ID NO: 27 und SEQ ID NO: 28.
7. Zur Identifizierung der Infektionsagenzien je nach ihrer Artzugehörigkeit, vorwiegend im urogenitalen Bereich, und zur spezifischen Erkennung von Bakterien Mycoplasma hominis ist eine beliebige von zwei Kombinationen verwendet. Diese Kombination schließt ein Differenzier-Oligonukleotid als Sonde und zwei spezifische Oligonukleotide als PCR-Primer ein. Eine dieser Kombinationen enthält ein Differenzier-Oligonukleotid SEQ ID NO: 73 und zwei spezifische Oligonukleotide SEQ ID NO: 29 und SEQ ID NO: 30. Die andere Kombination enthält das Differenzier-Oligonukleotid SEQ ID NO: 74 und zwei spezifische Oligonukleotide SEQ ID NO: 31 und SEQ ID NO: 32. Die dritte Kombination umfasst das Differenzier-Oligonukleotid SEQ ID NO: 75 und zwei spezifische Oligonukleotide SEQ ID NO: 33 und SEQ ID NO: 34.
8. Zur Identifizierung der Infektionsagenzien je nach ihrer Artzugehörigkeit, vorwiegend im urogenitalen Bereich, und zur spezifischen Erkennung von Bakterien Gardnerella vaginalis ist eine beliebige von zwei Kombinationen verwendet. Diese Kombination schließt ein Differenzier-Oligonukleotid als Sonde und zwei spezifische Oligonukleotide als PCR-Primer ein. Eine dieser Kombinationen enthält ein Differenzier-Oligonukleotid SEQ ID NO: 76 und zwei spezifische Oligonukleotide SEQ ID NO: 35 und SEQ ID NO: 36. Die andere Kombination enthält das Differenzier-Oligonukleotid SEQ ID NO: 77 und zwei spezifische Oligonukleotide SEQ ID NO: 37 und SEQ ID NO: 38. Die dritte Kombination umfasst das Differenzier-Oligonukleotid SEQ ID NO: 78 und zwei spezifische Oligonukleotide SEQ ID NO: 39 und SEQ ID NO: 40.
9. Zur Identifizierung der Infektionsagenzien je nach ihrer Artzugehörigkeit, vorwiegend im urogenitalen Bereich, und zur spezifischen Erkennung von Bakterien Streptococcus agalactiae ist eine beliebige von zwei Kombinationen verwendet. Diese Kombination schließt ein Differenzier-Oligonukleotid als Sonde und zwei spezifische Oligonukleotide als PCR-Primer ein. Eine dieser Kombinationen enthält ein Differenzier-Oligonukleotid SEQ ID NO: 79 und zwei spezifische Oligonukleotide SEQ ID NO: 41 und SEQ ID NO: 42.
10. Zur Identifizierung der Infektionsagenzien je nach ihrer Artzugehörigkeit, vorwiegend im urogenitalen Bereich, und zur spezifischen Erkennung von Bakterien Streptococcus agalactiae und/oder Streptococcus pyogenes ist eine beliebige von zwei Kombinationen verwendet. Diese Kombination schließt ein Differenzier-Oligonukleotid als Sonde und zwei spezifische Oligonukleotide als PCR-Primer ein. Eine dieser Kombinationen enthält ein Differenzier-Oligonukleotid SEQ ID NO: 80 und zwei spezifische Oligonukleotide SEQ ID NO: 43 und SEQ ID NO: 44.
11. Zur Identifizierung der Infektionsagenzien je nach ihrer Artzugehörigkeit, vorwiegend im urogenitalen Bereich, und zur spezifischen Erkennung von Viren Human Herpesvirus 1 und/oder Human Herpesvirus 2 ist eine beliebige von zwei Kombinationen verwendet. Diese Kombination schließt ein Differenzier-Oligonukleotid als Sonde und zwei spezifische Oligonukleotide als PCR-Primer ein. Eine dieser Kombinationen enthält ein Differenzier-Oligonukleotid SEQ ID NO: 81 und zwei spezifische Oligonukleotide SEQ ID NO: 45 und SEQ ID NO: 46. Die andere Kombination enthält das Differenzier-Oligonukleotid SEQ ID NO: 82 und zwei spezifische Oligonukleotide SEQ ID NO: 47 und SEQ ID NO: 48.
12. Zur Identifizierung der Infektionsagenzien je nach ihrer Artzugehörigkeit, vorwiegend im urogenitalen Bereich, und zur spezifischen Erkennung von Viren Human Herpesvirus 4 ist eine beliebige von zwei Kombinationen verwendet. Diese Kombination schließt ein Differenzier-Oligonukleotid als Sonde und zwei spezifische Oligonukleotide als PCR-Primer ein. Eine dieser Kombinationen enthält ein Differenzier-Oligonukleotid SEQ ID NO: 83 und zwei spezifische Oligonukleotide SEQ ID NO: 49 und SEQ ID NO: 50. Die andere Kombination enthält das Differenzier-Oligonukleotid SEQ ID NO: 84 und zwei spezifische Oligonukleotide SEQ ID NO: 51 und SEQ ID NO: 52. Die dritte Gruppe umfasst das Differenzier-Oligonukleotid SEQ ID NO: 85 und zwei spezifische Oligonukleotide SEQ ID NO: 53 und SEQ ID NO: 54.
13. Zur Identifizierung der Infektionsagenzien je nach ihrer Artzugehörigkeit, vorwiegend im urogenitalen Bereich, und zur spezifischen Erkennung von Viren Human Herpesvirus 5 ist eine beliebige von zwei Kombinationen verwendet. Diese Kombination schließt ein Differenzier-Oligonukleotid als Sonde und zwei spezifische Oligonukleotide als PCR-Primer ein. Eine dieser Kombinationen enthält ein Differenzier-Oligonukleotid SEQ ID NO: 86 und zwei spezifische Oligonukleotide SEQ ID NO: 55 und SEQ ID NO: 56. Die andere Kombination enthält das Differenzier-Oligonukleotid SEQ ID NO: 87 und zwei spezifische Oligonukleotide SEQ ID NO: 57 und SEQ ID NO: 58.

**Tabelle 1-1. Spezifische Oligonukleotide (Teil 1)**

| ID SEQ Nr. | Erreger | Nukleotidensequenz | |
|---|---|---|---|
| 1 | Chlamydia trachomatis | CCATTAGCAGAAGAAGTTCGCCGATTAGC | 159 |
| 2 | Chlamydia trachomatis | CTCTTTTCGTTGTAATGCAATTTGACTGGT | |
| 3 | Chlamydia trachomatis | GCTAGCTAAAGTTGCGAAACTCGTAGTTGC | 200 |
| 4 | Chlamydia trachomatis | GCGATCCATCCCCGATCCTTAGC | |
| 5 | Neisseria gonorrhoeae | ATCACTTCCCTGAACCGCGTGC | 138 |
| 6 | Neisseria gonorrhoeae | CCACGCCCTGAGGAGGAACAGC | |
| 7 | Neisseria gonorrhoeae | TGTACGGCGAAGTCAAAGCTGGTGT | 155 |
| 8 | Neisseria gonorrhoeae | CTTAAAGCCGATAAACGAGCCGAAATC | |
| 9 | Neisseria gonorrhoeae | GGTTCGATCCCGTTTGCCTCCA | 118 |
| 10 | Neisseria gonorrhoeae | TCCAATTTGTTAAAGATCGATGCGTCGT | |
| 11 | Trichomonas vaginalis | GGTAGGTGAACCTGCCGTTGGATCA | 154 |
| 12 | Trichomonas vaginalis | CGTTCTTCATCGTGTGAGGAGCCAAG | |
| 13 | Trichomonas vaginalis | GACGCACTCATGACGAACGAAGAAGG | 124 |
| 14 | Trichomonas vaginalis | TTACGCTTGGAGAGGACATGAACTTCG | |
| 15 | Ureaplasma urealyticum\ parvum | GTGACCGTCCTATCCAAGTTGGATCACA | 234 |
| 16 | Ureaplasma urealyticum\ parvum | CGTTAACTAAGCCGTTTACACCTCAAACTTC | |
| 17 | Ureaplasma urealyticum\ parvum | GGACGTCACACACCTATCGTTTCAGG | 157 |
| 18 | Ureaplasma urealyticum\ parvum | CAATCGCAACTGGGGCAATTAATTCTAC | |
| 19 | Candida albicans | CCTGCGGAAGGATCATTACTGATTTGC | 195 |
| 20 | Candida albicans | CATCGATGCGAGAACCAAGAGATCC | |

**Tabelle 1-2. Spezifische Oligonukleotide (Teil 2).**

| ID SE Q NO | Erreger | Nukleotidensequenz | |
|---|---|---|---|
| 21 | Candida albicans | CGCTGGGTTTGGTGTTGAGCAATAC | 120 |
| 22 | Candida albicans | GACGTTACCGCCGCAAGCAATG | |
| 23 | Mycoplasma genitalium | TCAAGGATAGCACCGGTGATTGGAG | 220 |
| 24 | Mycoplasma genitalium | AGAACTGTTCAAAAACATTCTTTCAGAACTG G | |
| 25 | Mycoplasma genitalium | CACCTGTTGAAATATGATTCAGCTCATGG | 193 |
| 26 | Mycoplasma genitalium | AAGCACCCTCTTCACTTACAAACCTACCAGT | |
| 27 | Mycoplasma genitalium | GAGCGAAATAAACAAATATGGATGAACGG | 267 |
| 28 | Mycoplasma genitalium | TTCATCAACATGCTTTTTAACTGCTGCTTC | |
| 29 | Mycoplasma hominis | TCCTTTCTACGGAGTACAACCTACGTTATGG | 254 |
| 30 | Mycoplasma hominis | GTAATCACGTCCTTCATCGACTTCCAGAC | |
| 31 | Mycoplasma hominis | GCTCACTTATTGAAATACGATACAGCTCATG G | 193 |
| 32 | Mycoplasma hominis | CACCTTCTTTTGTTACATATCTTCCAGTTCCT TC | |
| 33 | Mycoplasma hominis | GTAATATTAGTTGTGGGTGTCAATGGTTCAG G | 125 |
| 34 | Mycoplasma hominis | GCCGCTGCTCTAAATGTATCACCTGC | |
| 35 | Gardnerella vaginalis | CACGCTCGGTTGAGTGTGGTTACTG | 152 |
| 36 | Gardnerella vaginalis | AACCAAAAAGCCTCAACCTGTAATCCAAG | |
| 37 | Gardnerella vaginalis | CATTTTGGTGGAGTCGCTTGATCG | 120 |
| 38 | Gardnerella vaginalis | GCATCCAACCAATACGCCCTTACAGTG | |
| 39 | Gardnerella vaginalis | GTTACCGTTGAAGACAACAACAAGTTTGG | 229 |
| 40 | Gardnerella vaginalis | CAATAATGAGCAATGGCTTACCAGACTTCATAAC | |
| 41 | Streptococcus agalactiae | AGCAAATGGCTCAAAAGCTTGATCAAGA | 163 |
| 42 | Streptococcus agalactiae | TTCAAATCATAAACTGTCTCAGGGTTGGC | |
| 43 | Streptococcus agalactiae\ pyogenes | GCTCAATTGCTTTTGATGCGTGTCGA | 242 |
| 44 | Streptococcus agalactiae\ pyogenes | TTGCCCCCAAAGCTACTATCATTACCAGC | |
| 45 | Human Herpesvirus 1\2 | ACRTCCTCCTGTTTTCGCTCGG | 165 |
| 46 | Human Herpesvirus 1\2 | CACGAACTCGGGGCCGTACTG | |
| 47 | Human Herpesvirus 1\2 | CGACAAGATGGCGAGCCACAT | 154 |
| 48 | Human Herpesvirus 1\2 | AGATCCACGCCCTTGATGAGCAT | |
| 49 | Human Herpesvirus 4 | CCAACCMGAAATTTGAGAACATTGCAGA | 147 |
| 50 | Human Herpesvirus 4 | CTCGCCTKAGGTTGTAAAGGGAGGT | |
| 51 | Human Herpesvirus 4 | TTAGAGACAACCAGTGGAGTCCGCT | 101 |
| 52 | Human Herpesvirus 4 | GCAGGGACCAAGACAGGTGAACC | |
| 53 | Human Herpesvirus 4 | AAATTCGTGTGAGATGGACATCCAG | 163 |
| 54 | Human Herpesvirus 4 | GGTTCAGTGGTGGCATTGTGCT | |
| 55 | Human Herpesvirus 5 | ATGAARTGTATTGGGCTAACTATGCAGA | 259 |
| 56 | Human Herpesvirus 5 | GCAGCCATTGGTGGTCTTAGGGAA | |
| 57 | Human Herpesvirus 5 | CGCRTACGAGACCCCGAGGTA | 145 |
| 58 | Human Herpesvirus 5 | GMCGATTGGGGAGCGACAAGACA | |

**Tabelle 2-1. Differenzier-Oligonukleotide (Teil 1)**

| ID SE Q Nr. | Erreger | Nukleotidensequenz |
|---|---|---|
| 59 | Chlamydia trachomatis | TGTTTTTAATTCTTCAATAACCACTGTTAATGATTG CTGG |
| 60 | Chlamydia trachomatis | GCCTTTAGCCGTTTTAGTAGCGACAACACTGGCG AGC |
| 61 | Neisseria gonorrhoeae | ACTAATAGAGAACGAGCAAGGCTTCAAAGTTTTC CTGATG |
| 62 | Neisseria gonorrhoeae | CCTCAGAAGGTCAAACGGGCAATACAGTTACTAA GGCCA |
| 63 | Neisseria gonorrhoeae | CTGTTTTTAGCAGCTTATTTTGATTTGCGAAGTAG AA |
| 64 | Trichomonas vaginalis | TTCATCAAAAACCAAGTCTCTAAGCAATGGATGTC TTG |
| 65 | Trichomonas vaginalis | ATTGAAATGGGAATGGTCGCTGAAAAGATGGGTG TTT |
| 66 | Ureaplasma urealyticum\ parvum | P- CGAAGACAAAGAACGTAAAGTTGCTTATGGACGT CG |
| 67 | Ureaplasma urealyticum\ parvum | AGCTGGTGTTGACTTAGTAATGCCTGGTGATGAC GTTGAA |
| 68 | Candida albicans | TCAACTTGTCACACCAGATTATTACTAATAGTCAA AACTT |
| 69 | Candida albicans | GGATCGCTTTGACAATGGCTTAGGTCTAACCAAA |
| 70 | Mycoplasma genitalium | GTTTGGTCTCACAACTAACACATTTGGTCAGTTTG TATCC |
| 71 | Mycoplasma genitalium | GTTAAACAAAACATCTTGCAAATTGATAGAAAAAA GG |
| 72 | Mycoplasma genitalium | CCTTATGAACAAGATAGTAAAACAGGACACTACA ATGAAC |
| 73 | Mycoplasma hominis | TTTATCTCTCGGTTCTTTGAAAACTGAATATCGAC ATTGA |
| 74 | Mycoplasma hominis | ACACACAGAAAATTCTATTGTTGTAAATGGCAAAG AA |
| 75 | Mycoplasma hominis | AAAAAATTAATTGACGAGGGTAAAAAAGTATTGAT |
| | | TGCTG |
| 76 | Gardnerella vaginalis | CAATAGTTAATGTTTTTAGATGAGTTTGTTGGGCA TGCTT |
| 77 | Gardnerella vaginalis | GTTTTGTGATCGTTTAGTGTGATGATTTATTATGT CTAGAGAG |
| 78 | Gardnerella vaginalis | GGAAATATAACCCTTATCAAAACGCATACCTTCAG TGAAGTCCAA |
| 79 | Streptococcus agalactiae | CAAAAGCTTGATCAAGATAGCATTCAGTTGAGAA ATATCA |
| 80 | Streptococcus agalactiae\ pyogenes | [?] GGAGCTAAGGTGATTAATATGAGCTTTGGTAATG CTGCAC |
| 81 | Human Herpesvirus 1\2 | GCGAATTCGAGATGCTGYTGGCCTTCATGACC |
| 82 | Human Herpesvirus 1\2 | CGAGTGCGAAAARACGTTCACCAAGCTGCTGCT |
| 83 | Human Herpesvirus 4 | TTGGGTCGCCGGTGTGTTCGTATATGGAGGTAGT |
| 84 | Human Herpesvirus 4 | GCTGTCGGCGTCCACTCTCTTTCCCCTTGT |
| 85 | Human Herpesvirus 4 | ACGGCTTGTCCCCACCCCATGGATTTCTATTG |
| 86 | Human Herpesvirus 5 | GTATGAGAACTACATTGTACCTGAGGATAAGCGG GAGA |
| 87 | Human Herpesvirus 5 | CGGTCTGCAGTGCGGCGTACAACGTGTGGA |

### Synthese von Sonden und Primer

Die Oligonukleotiden werden mittels einer automatischen Syntheseeinrichtung (z. B., Baureihe Gene Assembler" von Pharmacia) nach dem genormten Amidophosphit-Verfahren zusammengesetzt. Um die Immobilisierung sicherzustellen, werden die Differenzier-Oligonukleotide auf der Chip-Oberfläche nach der 5'- oder 3'-Endestelle (terminus) modifiziert. Z. B., wird die endständige 5'-Phosphatgruppe während der Synthese eingeführt. Ein Oligonukleotid aus dem Paar der spezifischen Oligonukleotide zur PCR-Durchführung wird nach der 5'-Endstelle mittels eines Markers modifiziert. Der Marker wird aus einer Gruppe gewählt, welche einen katalytischen, einen Ligand-, einen Fluoreszent- und einen radioaktiven Marker einschließt. Der Marker wird mittels chemischer Verfahren bzw. enzymatisch eingeführt.

### Vorbereitung des biologischen Mikrochips (Oberflächenmodifizierung)

Die Matrize weist Oberflächenaminogruppen auf. Die Matrize wird mittels einer Lösung von 3-Aminopropyltrietoxysilan (APTES) mit unterschiedlichen Lösungsmitteln je nach dem Trägertyp. Die Glasträger werden z. B. in der 3-Aminopropyltrietoxysilan-Lösung im Azeton oder im Äthanol inkubiert. Die APTES-Konzentration variiert umfangsmäßig im Bereich von 0,1 bis 10 %. Wird die Reaktion im Azeton durchgeführt. Die Gläser werden dreimal je 5 Minuten lang mit Azeton, danach zweimal je 3 Minuten lang mit Äthanol abgespült und bei Temperatur von 120 - 130 °C 20 Minuten lang durchgeglüht.

### Das Auftragen von Sonden

Die Reaktionsmischung enthält 1 µM von Oligonukleotid und 60 mM von Hydrochlorid 1-Äthyl-3-(3-Dimethylaminopropyl)-Karbodümid in 0,1 M von 1-Methylimidazol, pH 7.0. Die Reaktionsmischung wird auf die Matrizeoberfläche mittels eines Roboter (Xpress Lane, USA) aufgetragen. Das Tropfenvolumen jeder Sonde beträgt mindestens 0,005 µl. Die Matrizen mit den darauf aufgetragenen Oligonukleotiden werden in einen abgedichteten Behälter gesetzt und bei Raumtemperatur in der feuchten Atmosphäre 1 Stunde lang gehalten. Danach wird 1 M im Shaker mit der NaCl-Lösung 10 Minuten lang und dann mit destilliertem Wasser gespült. Der Biochip wird dann abgetrocknet und bei Raumtemperatur gelagert. Die Oligonukleotid-Konzentration im Tropfenvolumen jeder Sonde beträgt von 0,5 bis 100 µM.

### DNS-Aussonderung und Vorbereitung des Materials zur Amplifikation

Die DNS-Aussonderung aus der biologischen Probe (Blut, Biopsie-Material, Speichel, Tränenabsonderungen, Harn, bronchoalveoläre Lavage, Rückenmarkflüssigkeit, Liquor usw.) wird unter Einsatz von landläufigen Methoden durchgeführt. Diese Methoden beruhen z. B. auf der Alkalilysis, Anwendung von Reinigungsmitteln, Proteinase K, Extraktion mittels Phenols, Chloroforms und/oder mittels der Phenol-Chloroformmischung bzw. auf der Reinigung auf Diatomeenerde oder Siliziumdioxid. Vor der DNS-Aussonderung wird die Probe mit der DNS der positiven Kontrollmittel ergänzt.

### Positive und negative Kontrollmittel (Positive and negative controls)

Im Rahmen dieser Erfindung werden die positiven Kontrollmittel im Diagnosesatz zur Bestätigung der Einhaltung von Optimalbedingungen bei der Durchführung von Prozeduren benutzt, die zur Identifikation von angemeldeten Pathogenorganismen, und zwar zur Aussonderung von DNS aus dem biologischen Material sowie für die Durchführung von PCR und Hybridisierung erforderlich sind. Als positives Kontrollmittel 1 dient die DNS mit der bekannten Nukleotidensequenz. Diese DNS kann sowohl die natürliche DNS von einem Organismus als auch eine künstliche Sequenz, z. B., die rekombinante DNS sein. Das kann auch ein Abschnitt dieser DNS sein, der anhand von PCR oder einem anderen Verfahren erhalten wurde. Die DNS vom positiven Kontrollmittel 1 wird anhand von asymmetrischer PCR unter Einsatz von spezifischen Oligonukleotiden ebenso wie die zu erforschenden Proben amplifiziert. Die spezifischen Oligonukleotide werden für die DNS-Amplifikation des positiven Kontrollmittels 1 so gewählt, dass die DNS-Amplifikation anhand von PCR unter den gleichen Bedingungen wie auch für die zu ermittelten Pathogenorganismen vorgenommen werden kann. Zur Identifikation der DNS des positiven Kontrollmittels 1 wird auf dem Chip das Differenzier-Oligonukleotid immobilisiert, welches die in der Einkettenform erzeugte DNS-Sequenz des positiven Kontrollmittels ergänzt. Als positives Kontrollmittel 2 wird auf dem Chip ein Oligonukleotid oder mehrere Oligonukleotide immobilisiert, die die spezifischen Oligonukleotide für PCR ergänzen. Diese werden mittels eines Markers aus der Gruppe gemäß "Sonden- und Primersynthese" nach der 5'-Endstelle (terminus) modifiziert.

Die negativen Kontrollmittel werden im Rahmen dieser Erfindung zur Bestätigung der Einhaltung der Optimalbedingungen bei der Hybridisierung verwendet. Als negatives Kontrollmittel wird auf dem Chip ein Oligonukleotid immobilisiert, welches teilweise die in der Einkettenform erzeugte DNS-Sequenz des positiven Kontrollmittels ergänzt. Es können auch Oligonukleotide sein, welche teilweise die spezifischen Oligonukleotide für die PCR ergänzen. Diese spezifischen Oligonukleotide sind mit dem Marker aus der Gruppe gemäß "Sonden- und Primersynthese" nach der 5'-Endstelle (terminus) modifiziert. Das Differenzier-Oligonukleotid dient als negatives Kontrollmittel. In der Sequenz des Differenzier-Oligonukleotids werden die Anzahl und die Lage der Nukleotide, die keiner DNS-Sequenz des positiven Kontrollmittels oder den spezifischen Oligonukleotiden für PCR entsprechen, so gewählt, dass dieses Differenzier-Oligonukleotid fähig ist, das inkomplette Duplex mit Einketten-DNS des positiven Kontrollmittels oder des spezifischen Oligonukleotids für PCR zu bilden und aufrechtzuerhalten. Und dies zwar bei Nichteinhaltung der Bedingungen für die Hybridisierung und das Auswaschen, nämlich wenn die Hybridisierung und das Auswaschen unter milderen Bedingungen gegenüber den Optimalbedingungen erfolgen. Als Beispiel, welches den Erfindungsgehalt umfasst, jedoch nicht einschränkt, sind in der Sequenzliste die spezifischen Oligonukleotide ID SEQ No: 88 und 89 angeführt. Sie verwenden die Plasmide pUC18 zur DNS-Amplifikation anhand von asymmetrischer PCR. Die Liste enthält auch Differenzieroligonukleotide ID SEQ No: 90 und 91, welche auf der Chip-Oberfläche immobilisiert und dementsprechend als positive und negative Kontrollmittel benutzt werden.

### Markerwahl

Es sind viele Patentschriften bekannt, in denen verschiedene Markertypen zum Aufbau der biologischen Chips beschrieben sind. Es ist eine gruppenweise Markerklassifikation bekannt. Diese Gruppen von Markern umfassen katalytische, Ligand-, Fluoreszent- oder radioaktive Moleküle [21, 22]. Die genannten Patentschriften sind als Literaturverweise angeführt. Das katalytische Molekül wird aus der Gruppe gewählt, welche Hämin, Zyankobalamin oder Flavin enthält. Das Ligand-Molekül wird aus der Gruppe gewählt, die Biotin, Dioxigenin oder Donitrobenzol enthält. Das fluoreszente Molekül wird aus der Gruppe mit FAM, TAMRA, Cy3, Cy5, Cy7, R6G, R110, ROX oder JOE gewählt.

### DNS-Amplifikation anhand von PCR

Die Einfaden-DNS-Abschnitte der typischen Sequenzen von Infektionsagenzien gemäß Tabelle 1 werden während der Reaktion der asymmetrischen PCR unter Einsatz von hochspezifischen Primer erzeugt.

### PCR-Bedingungen

Die PCR-Mischung enthält einen Reaktionspuffer, z. B., von 10 bis 80 mM tris-HCl pH 8,8, bis zu 50 mM KCI und/oder bis zu 25 mM (NH₄)₂SO₄, 0,1 % Triton X-100, oder 0,1 % Tween 20, oder 0,8 % Nonidet P40, von 1 mM bis 5 mM, optimal von 1,5 mM bis 2,5 mM, MgCl₂ oder MgSO₄ von 10 µg bis 300 µg, optimal von 50 µg bis 200 µg, DNS der zu analysierenden Probe und mindestens ein Paar von spezifischen Oligonukleotide-Primer für jedes Target. Die Gesamtanzahl von spezifischen Oligonukleotide-Paaren kann von 1 bis 29 variieren und zwar in Kombinationen zur Identifikation von mindestens einem Infektionsagens in Form von Pathogen-Mikroorganismen und/oder Pilze und/oder Viren. Die PCR-Mischung enthält 1 bis 20 Einheiten, optimal 6 bis 14 Einheiten, am besten jedoch 8 bis 12 Einheiten der Taq-Polymerase-Aktivität. Die PCR-Mischung kann auch Reagenzien enthalten, welche die Spezifität und die Effektivität von PCR erhöhen. Es handelt sich z. B. um Betain, Ektain und seine Derivate, Trehalose, Dimethylsulfoxid und Glyzerin.

Die Konzentration des unmarkierten spezifischen Oligonukleotides in der PCR kann 0,025 µM bis 0,2 µM, optimal 0,05 µM bis 0,1 µM betragen. Die Konzentration des markierten spezifischen Oligonukleotides in der PCR beträgt 0,125 µM bis 1 µM, optimal jedoch 0,25 µM bis 0,5 µM. Das Verhältnis des unmarkierten Oligonukleotides zum markierten beträgt 1/2 bis 1/50, optimal 1/5 bis 1/10. Die Menge der Reaktionmischung kann 5 µl bis 200 µl, optimal 20 µl bis 50 µl betragen. Die Reaktion kann unter Mineralöl oder ohne Mineralöl, je nach der Konstruktion eines DNS-Verstärkers, durchgeführt werden. Die PCR wird unter Einsatz von DNS-Verstärker (z. B. Tercik, DNA Technology, Russland, oder Mastercycler, Eppendorf, Deutschland), unter den in Tabelle 2 angeführten Bedingungen vorgenommen.

**Tabelle 2. PCR-Bedingungen**

| Programmschritt | Temperatur, °C | Inkubationszeit, s | Zykluszahl |
|---|---|---|---|
| 1 | 94 ± 2 | 180 ± 120 | 1 |
| 2 | 94 ± 2 | 20 ± 10 | Von 35 bis 45 |
| 3 | 63 ± 3 | 20 ± 10 | |
| 4 | 72 ± 2 | 30 ± 10 | |
| 5 | 72 ± 2 | von 180 bis 300 | 1 |

### Hybridisierung

Die markierten PCR-Produkte werden mit DNS-Sonden (Differenzier-Oligonukleotiden) hybridisiert. Diese werden auf einem Biochip in einem extra ausgewählten Puffer immobilisiert.

### Hybridisierungsbedingungen:

Die Hybridisierung wird in einem Puffer mit 0,5 x - 5 x SSC, 0,1 % SDS, bis 25 % Formamid und/oder bis zu 10 mM EDTA im Laufe von 1 Stunde bei 35 - 50 °C vorgenommen. Danach wird der Chip bei der Raumtemperatur im Orbitalshaker 5 bis 10mal mit Lösungen von 0,5 x - 5 x SSC, 0,1 % SDS gespült. Werden die Fermente zur Demonstration der Hybridisierungsergebnisse eingesetzt, dann werden die 3 letzten Spülungen mit der SSC-Lösung ohne SDS vorgenommen.

### Durchführung der Peroxydase-Reaktion

Sind die PCR-Produkte mit Biotin markiert, so wird die Reaktion zur weiteren Identifikation unter Einsatz von Streptavidin-Peroxidase- bzw. Streptavidin-Phosphatase-Konjugat durchgeführt.

### Biochip-Struktur

Der biologische Mikrochip zur Identifizierung der Infektionsagenzien je nach ihrer Artzugehörigkeit, vorwiegend im urogenitalen Bereich, kann sowohl als ein Einzelchip als auch in Form eines Multichips ausgeführt werden. Der biologische Mikrochip wird auf der Oberfläche eines allgemeinen Trägers mit N Einzelchips realisiert. Der Arbeitsbereich des Einzelchips enthält immobilisierte Cluster von Differenzieroligonukleotide-Sonden mit einer oder mehr Sequenzen aus der Gruppe SEQ ID NO:59-87. Der Mikrochip-Träger kann als flache Platte oder Folie ausgeführt werden.

Es ist die Ausführung eines Multichips in Form von rechteckigen Flachbetten [15], rechteckigen Platten [16] oder einer flachen Scheibe [17] bekannt. Die bevorzugte Form des Multichips ist eine Platte mit unterschiedlicher Stärke. Im Rahmen dieser Erfindung wird die Multichipform aufgrund der vorhandenen Vorinformationen über den Typ des Multichipmaterials, über das voraussichtliche Verfahren der Oberflächenmodifizierung, über das vermutliche Verfahren der Analysedatenverarbeitung und die Wahl der Ausrüstung zur Informationserfassung und -bearbeitung gewählt. Der Multichip kann als ein Rechteck, ein Quadrat, eine Scheibe oder ein Polygon ausgeführt werden. Die Trägerstärke kann 0,5 bis 5 mm betragen.

In Fig. 1 sind die eventuellen Anordnungen der Multichipelemente auf der Trägeroberfläche abgebildet, ohne dass ein gemeinsamer Bereich gebildet wird.

In den Fig. 1 (a) - (d) ist die Zonenverteilung auf dem Träger des Multichips für verschiedene Anordnungsmöglichkeiten abgebildet. Im Allgemeinen enthält der Multichip einen Träger 100. Auf seiner Oberfläche werden N vom Träger trennbare Einzelchips in Abschnitten 102 angeordnet. Die Anzahl N der Einzelchips beim Multichip darf mindestens 2 betragen. Jeder Abschnitt 102 wird einem bestimmten Einzelchip zugeordnet. Auf der Oberfläche jedes Abschnittes 102 wird eine Zonengruppe gebildet, und zwar: a) Arbeitszone des Chips zur Sondeneinführung 103, b) eine Zone zur Einführung des ersten Identifizierungsmerkmals 104 und optional des zweiten Identifizierungsmerkmals 105. Dabei besteht die Arbeitszone des Einzelchips aus der Clustergruppe 106. Jeder der Cluster wird durch seine eigene Sonde gebildet. Die Sonden werden zwecks Anordnung auf der Chiparbeitsfläche gewählt. Die Ausnahme davon sind die Sonden für die Probendiagnose. Die Sondengruppe umfasst solche Sonden, die den positiven oder negativen Kontrollmitteln angehören. Sie umfasst auch alle Sonden, die als Marker ausgeführt sind, z. B., Marker zur Identifizierung der Chipposition. Zusätzlich steht jeder Abschnitt im Kontakt mit der Zone 107. Die Zone 107 kontaktiert die Grenzen von mindestens zwei Einzelchips, welche an die Außenkontur jedes Abschnitts anstoßen. Die Zone 107 wird mit Aussparungen 108 und/oder mit durchgehenden Bohrungen 109 zwischen den Chips versehen. Die durchgehenden Bohrungen 109 können in Form einer Lochung, von Schlitzen, Lücken oder Abständen zwischen den Seitengrenzen der Chips ausgeführt werden. Die Fig. 1 (c) enthält eine Ausführung eines Multichips mit der Kombination von Aussparungen und durchgehenden Bohrungen zwischen den Chips und mit der Anordnung von zwei Identifizierungsmerkmalen. In Fig. 1 (d) ist eine Ausführung von einem Multichip mit einem Identifizierungsmerkmal enthalten. Die Aussparungen und/oder die durchgehenden Bohrungen zwischen den Chips vereinfachen die Trennung der Einzelchips 110 vom Träger 100 oder voneinander. Die Möglichkeit der einfachen Trennung der Einzelchips vom Multichip ermöglicht es, ein konsequentes Screening unter Einsatz von einem Multichip durchzuführen, bei dem alle Einchips unter gleichen Bedingungen hergestellt wurden. Das ermöglicht es, die einzelnen Chips im Laufe der aufeinander folgenden Diagnosen, z. B. während der Krankenbehandlung miteinander zu vergleichen. Dabei wird während der konsequenten Selektion der Proben im Laufe der Diagnosestellung konsequent mindestens ein Einzelchip aus der Gruppe von Einzelchips getrennt. Dieser Einzelchip wird zur Hybridisierung mit den PCR-Produkten in einer individuellen Zelle benutzt.

Die Fig. 1 (a) enthält eine Ausführung eines Multichips, wobei auf der Multichipoberfläche nur Aussparungen ohne durchgehende Bohrungen zwischen den Chips gebildet werden. Diese Ausführung enthält auch die Anordnung von zwei Identifizierungsmerkmalen. Diese Lösung ist bei der Durchführung von Massenscreenings bevorzugt, wenn alle Chips eines Multichips einer Parallelbearbeitung unterzogen werden müssen, und wenn sie nach der vorgenommenen Diagnose gleichzeitig vom Multichip getrennt werden können.

In der vorgeschlagenen Erfindung wird das Problem einer Kreuzkontamination durch die Kombination von Aussparungen und durchgehenden Bohrungen beseitigt. Die durchgehenden Bohrungen sind in Form von Lochung, Schlitzen, Lücken bzw. Abständen zwischen den Seitengrenzen der Chips ausgeführt. In diesem Fall werden die Überschüsse der Hybridisierungsmischung in die Aussparungen oder in die durchgehenden Bohrungen ablaufen. Beim Abgießen oder Pressen des Multichip-Trägers können zusätzlich 0,1 bis 5 mm tiefe Vertiefungen auf der Oberfläche des Arbeitsbereichs jedes Einzelchips ausgeführt werden. In diesen Vertiefungen können die Sonden der Einzelchips angeordnet werden. Die Vertiefungen können auch zur Einführung einer Hybridisierungsmischung in die Arbeitszone verwendet werden. Das ermöglicht es, die Einzelchips bzw. eine Gruppe von Einzelchips vom Multichipkörper problemlos zu trennen, um den Scanning-Vorgang mit einem beliebigen Scanner durchzuführen. Der Multichip kann auch vollständig auf einem Flachbettscanner eingescannt werden. Dabei werden die Angaben aller Identifizierungsmerkmale abgelesen.

Die Fig. 2 enthält Beispiele für Multichipträger-Querschnitte bei verschiedenen Ausführungen der Aussparungen: a) dreieckige Aussparung 120, b) Kugelaussparung 121, c) rechteckige Aussparung 122, d) Polygonaussparung 123, e) Ausführung mit der Aussparung an beiden Seiten des Trägers 124. Dabei können die Aussparungen, vorwiegend beim Pressen oder Abgießen, sowohl an einer als auch an beiden Seiten des Multichips angeordnet werden. Die Stärke des Trägers wird im Bereich 0,5 bis 5 mm gewählt. Dabei wird das Verhältnis der Trägerschichtstärke zur Aussparungstiefe im Multichip aufgrund der mechanischen und physikalischen Parameter des gewählten Materials (Härte, Brüchigkeit) für den jeweiligen Multichip gewählt. Dieses Verhältnis kann 2:1 bis 100:1 betragen. Wird die Kombination von Aussparungen und durchgehenden Bohrungen in der Zone 107 benutzt, so wird das Verhältnis zwischen den Längen der Aussparungen und durchgehenden Bohrungen zwischen den Chips im Bereich 1:10 bis 10:1 gewählt. Das Verhältnis zwischen den Breiten der Aussparung und der durchgehenden Bohrungen kann im Bereich 1:10 bis 10:1 gewählt werden. Die Aussparungsbreite wird im Bereich 0,1 bis 5 mm gewählt.

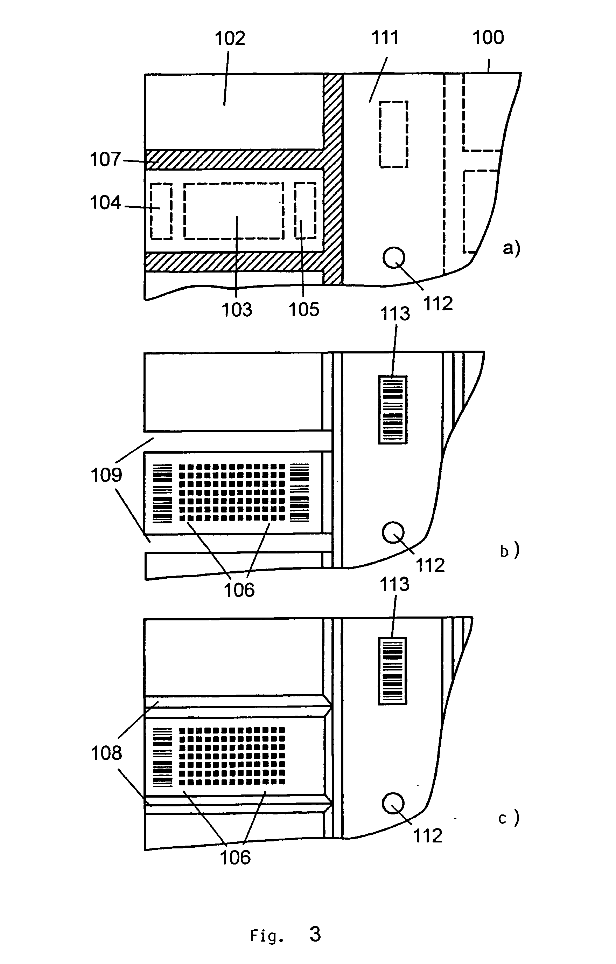

In Fig. 3 sind die Anordnungsmöglichkeiten der Hauptelemente des Multichips auf der Trägeroberfläche abgebildet. Die gemeinsame Zone 111 wird auf dem Träger eingerichtet. Die gemeinsame Zone 111 des Trägers des Multichips 100 kann seitlich, oberhalb, unterhalb oder mittig in Bezug auf die Chip-Reihe liegen. Diese Zone kann als ein Rechteck ausgeführt werden oder z. B. eine Kreuzform aufweisen. Die gemeinsame Zone hat zwei Funktionen. Sie dient zum einen zur Ausführung von Einstell-, Justier- und Befestigungslöchern 112. Sie vereinfachen die Befestigung von vorgefertigten Multichips bei der Modifizierung der Oberflächen, bei zahlreichen Spülungen und Oberflächenreinigungen vor der Einführung der Sonden. Die zweite Funktion der gemeinsamen Zone ist die Aufnahme eines dritten Identifizierungsmerkmals 113. Dieses Identifizierungsmerkmal 113 kann die Informationen enthalten, welche für die nachfolgende Auswertung der Diagnosedaten während des Parallel- oder der aufeinander folgenden Screenings von Biopolymeren nutzbar sein können. In Fig. 3(a) ist die Zonenverteilung auf dem Multichip-Träger abgebildet. Im allgemeinen Fall enthält der Multichip einen Träger 100. Auf seiner Oberfläche werden N Chips in Abschnitten 102 angeordnet. Die Oberfläche jedes Abschnitts dient zur Bildung von Zonengruppen: a) Arbeitszone des Chips zur Sondeneinführung 103, b) Zone zur Einführung des ersten Identifizierungsmerkmals 104 und zur optionalen Einführung des zweiten Identifizierungsmerkmals 105. Dabei besteht die Arbeitszone des Einzelchips aus der Clustergruppe 106. Jeder der Cluster wird durch seine eigene Sonde gebildet. Die Zusammensetzung der in der Chiparbeitszone angeordneten Cluster wird aus der folgender Gruppe gewählt: Sondencluster, Cluster der positiven Kontrollmittel, Cluster der negativen Kontrollmittel. Die Anzahl der in der Arbeitszone jedes Einzelchips angeordneten Cluster kann von 2 bis 1000 betragen. Bei preiswerten Chips können in der Arbeitszone jedes Einzelchips 15 Cluster angeordnet werden.

Die Anzahl der eingeführten Sonden von einem Typ des Differenzier-Oligonukleotides auf der Oberfläche jedes Clusters beträgt 1 bis 50. Bei preiswerten Chips sollten auf der Oberfläche jedes Clusters bevorzugt 4 bis 9 eingeführte Sonden von einem Typ des Differenzier-Oligonukleotides angeordnet werden.

Neben den Sonden für die Probendiagnose umfasst die Gruppe der auf der Oberfläche der Arbeitszone angeordneten Sonden auch andere Sondengruppen. Das sind unter anderem die Sonden der positiven oder negativen Kontrollmittel. Eine Sondengruppe kann auch als Marker ausgeführt werden, z. B. als Marker, die die Chipposition identifizieren. Das Verhältnis zwischen der Anzahl der Cluster mit Sonden und der Anzahl der Cluster mit den darin angeordneten positiven oder negativen Kontrollmitteln, wird im Bereich 1:1 bis 500:1 gewählt. Jeder Abschnitt 102 kontaktiert mit einer Zone 107, die ihrerseits die Grenzen von mindestens zwei Einzelchips (die an die Außenkontur jedes Abschnitts angrenzt) und die Grenze der gemeinsamen Zone des Multichips berührt. In der Zone 107 werden Aussparungen 108 und/oder durchgehende Bohrungen 109 zwischen den Chips ausgeführt. In Fig. 3(6) ist eine Ausführung eines Multichips mit einer Kombination von Aussparungen und durchgehenden Bohrungen zwischen den Chips sowie mit der Anordnung von drei Identifizierungsmerkmalen abgebildet. Die Aussparungen und/oder die durchgehenden Bohrungen (Abstände zwischen den Chips) stellen die Abtrennung der Chips 110 vom Träger 100 oder voneinander sicher. In Fig. 3(c) ist eine Ausführung eines Multichips mit Aussparungen zwischen den Chips sowie mit der Anordnung von zwei Identifizierungsmerkmalen abgebildet. Die einzelnen Mikrochips werden vom Träger abgetrennt. Die Trennung erfolgt mittels Abbrechens, Abschneidens oder Abhackens.

Es ist bekannt [16], dass der Multichip-Träger aus Schießbaumwolle, Glas, Kunststoff, Teflon, Metall sowie aus ihren Kombinationen gefertigt ist. Es wurde jedoch bereits erwähnt, dass nicht alle genannten Werkstoffe fähig sind, die Oligonukleotid-Sonden kovalent zu immobilisieren. Als Material zur Fertigung von Multichipträgern werden Polymere, Glas, Keramik oder ihre Kombinationen vorgeschlagen.

Für die Glasträger wurden viele Verfahren der Oberflächenmodifizierung zur nachfolgenden Immobilisierung der Sonden entwickelt. Jedoch ist die massenweise Anwendung der Multichips aus Glas durch die Brüchigkeit dieses Materials eingeschränkt. Sie erschwert ihre Herstellung und den Transport. Die Ausführung von zweiseitigen Aussparungen oder Aussparungen und durchgehenden Bohrungen zwischen den Chips ermöglicht es, die Glasplatten voneinander zu trennen, ohne den Chipträger zu beschädigen.

Polymere sind nicht brüchig und können in unterschiedlichen Verfahren gefertigt werden, darunter auch Stanzen, Pressen, Abgießen, in einer mechanischen Bearbeitung. Es können folgende Polymere angewendet werden: Polymethylmethakrylat, Polybutylmethakrylat, Polyvinylchlorid, Polykarbonat, Copolymere von Methylmethakrilat und/oder Copolymere von Butylmethakrylat mit anderen Monomeren, z. B. Styrol, Vinylzyanid usw. Bevorzugt wird Polymethylmethakrylat angewendet. Es weist eine niedrigere Stufe von Autofluoreszenz auf, ist durchsichtig und kann die Grundlage für einen Verbundträger darstellen. Polyvinylchlorid ermöglicht es, die Oberfläche effektiv zu gestalten und die Basis für die Fertigung von Einzelmultichips zu erhalten.

Wird der Multichip als ein Rechteck ausgeführt, dann sollte das Verhältnis zwischen der Breite und der Länge des Multichips vorzugsweise im Bereich 1:1 bis 1:50 betragen. Bei manchen Ausführungen sollten die Abmessungen von Multichip vorzugsweise innerhalb der Dimensionen von genormten Papierbögen, z. B., DIN A4, gewählt werden, da die Scanner für solche Abmessungen ausgelegt sind. Unter Berücksichtigung des hohen Auflösungsvermögen der modernen Scanner und der perfekten Verfahren der Einführung von Sonden in die Chipoberfläche ist es von Vorteil, nicht nur die genormten Abmessungen von 25 mm x 75 mm für Einzelchips, sondern auch andere Maße zu verwenden. Z. B. können um die Kosten im Zusammenhang mit der Vorbereitung von Einzelchips zum Einbau in den Multichip zu minimieren, Chips mit folgenden Abmessungen verwendet werden: 12,5 mm x 75 mm, Minichips mit den Abmessungen 25 mm x 37,5 mm oder 12,5 mm x 37,5 mm.

Eine Ausführungsform dieser Erfindung stellt einen individuellen biologischen Mikrochip dar, der in einen Satz für die Diagnose eingeschlossen werden kann. Er kann als ein Einzelchip oder als Bestandteil eines Multichips ausgeführt werden. Dieser Chip enthält 15 Gruppen (Cluster) von DNS-Sonden. Jede dieser Gruppen ist durch ein der 15 immobilisierten Oligonukleotide repräsentiert (Fig. 6 und Tabelle 1). Der Chip enthält 15 Cluster von individuellen Differenzier-Oligonukleotiden, welche mittels 4 oder 9 Schritte aufgetragen wurden. Davon sind 12 Oligonukleotide-Cluster für ein oder zwei der 15 Infektionsagenzien aus der Tabelle 1, 2 Cluster für ein positives Kontrollmittel und 1 Cluster für ein negatives Kontrollmittel bestimmt. Das resultierende Hybridisierungsbild für das Versuchsmuster wird mit positiven und negativen Kontrollmitteln verglichen. Das ermöglicht es, eine wahrheitsgemäße Identifizierung nach der Artzugehörigkeit vorzunehmen.

### Identifikation der Multichip-Daten

Es gibt mehrere Beispiele für die Anwendung der Identifizierungsmerkmale zur Identifikation von Biochips. Es ist z. B. eine Erfindung [18] bekannt, wobei der Chip zwei Strichkodes zur Identifikation von zwei Arbeitszonen aufweist. Es ist eine Erfindung [16] bekannt, worin jede einzelne Zone mit den ihr zugeordneten kodierten Daten in Form von einem Identifizierungsmerkmal vom Multichip getrennt und individuell eingesetzt ist. Es ist eine Erfindung [19] bekannt, welche die computergestützte Anwendung von Identifizierungsmerkmalen zur Qualitätskontrolle bei der Chip-Fertigung offenbart.

Der Unterschied der vorgeschlagenen Erfindung gegenüber dem bekannten Stand der Technik besteht darin, dass der Einzelchip sowie der Multichip, je nach der medizinischen Diagnoseart, mindestens eine zwei- oder dreistufige Identifikation der Daten über den multifunktionellen Multichip aufweist, wobei der Multichip N Einzelchips einschließt. Dafür wird auf jedem Einzelchip mindestens ein erstes Identifizierungsmerkmal angeordnet, welches für die Identifikation der Daten über den biologischen Forschungsgegenstand konzipiert ist. Es kann darauf auch ein zweites Identifizierungsmerkmal optional angeordnet werden. Das zweite Identifizierungsmerkmal enthält die Daten über die Fertigung, den Typ und die Parameter der Sonden. Dabei enthält der Mehrfunktions-Multichip im Befestigungsbereich aller Chips zusätzlich ein drittes Identifizierungsmerkmal, welches mit dem zweiten Identifizierungsmerkmal identisch sein kann. Dabei ist das erste Identifizierungsmerkmal mit einem ersten Kode verbunden. Das zweite Identifizierungsmerkmal ist mit einem zweiten Kode verbunden, welcher mit dem ersten Kode nicht identisch ist. Die genannten Kodes bilden einzigartige (unikale) N Kodepaare, welche den Mehrfunktionschip kennzeichnen. Das Identifizierungsmerkmal kann in Form von Nummern- und/oder Buchstabenzeichen bzw. Strichkode oder ihren Kombinationen ausgeführt werden. Sie haben die Funktion von einem Kode.

Als Beispiel zu dieser Erfindung sind weiter unten Parameter angeführt, welche als Parameter des ersten Identifizierungsmerkmals bei der Feststellung oder der Diagnose von Säugetiererkrankungen eingeführt werden können. Dieses Beispiel umfasst den Erfindungsgehalt, beschränkt ihn jedoch nicht.

Das erste Identifizierungsmerkmal des Einzelchips kann folgende Daten einschließen: a) Krankheitskode, b) Kode der Probenquelle (Blut, Lymphe, Abradat, Speichel usw.), c) Patientenkode oder Datenkode, welcher den Patienten identifiziert (Name, Alter), d) Kode der medizinischen Anstalt und/oder der Versicherungsgesellschaft, e) Diagnosedatum. Die Parameter des zweiten und des dritten Identifizierungsmerkmals identifizieren den Multichip können ihrerseits folgende Daten enthalten:
a) Kode der Chargennummer und/oder des Herstellungsdatums,
b) Herstellerkode,
c) Kode, welcher das Verfahren der Oberflächenmodifizierung identifiziert,
d) Kode des Screeningstyps (konsekutiv, parallel), e) Kode, welcher die Einzelchipanzahl kennzeichnet,
e) Kode, welcher die Clusteranzahl kennzeichnet.

Solche Information kann bei der Ermittlung der Qualitätsmerkmale eines Multichips und auch von seinen Bestandteilen benutzt werden. Dabei muss die Kombination des ersten und des zweiten Kodes oder des ersten, des zweiten und des dritten Kodes einen einzigartigen Kode ergeben, welcher nur einem Multichip entspricht. Die Verwendung dieses einzigartigen Kodes stellt eine schnelle Suche der erforderlichen Informationen über alle Proben beim Parallelscreening bzw. über die aufeinander folgenden Messungen sicher. Die Suche erfolgt quer über alle Datenbanken.

### Hard- und Software zur Identifikation der Diagnosedaten

Um die Möglichkeiten des Multichips im Zusammenhang mit der Durchführung von Parallel- und konsekutiven Screenings der biologischen Gegenstände zu realisieren, ist eine Gesamtheit von Hard- und Software zur Bearbeitung der resultierenden Daten, ihrer Identifikation und ihrer Aufnahme in die Datenbank erforderlich.

In Fig. 4 ist ein Übersichtsschaltplan der Anlage zur Durchführung von Parallel- und konsekutiven Screenings der biologischen Gegenstände abgebildet. Diese Anlage beinhaltet einen PC 201, an den verschiedene Einrichtungen angeschlossen werden können, und zwar eine Anzeige 202, ein Drucker 203 und eine Einrichtung zum Einscannen der Multichips oder der Einzelchips. Diese Einrichtungen können aus folgender Gruppe gewählt werden: Flachbettscanner 210, digitale CCD-Kameras 211, Scanner 212 der Einzelchips 110, Scanner 213 für die Multichips auf der Scheibe 140 und Handscanner 214. Die Daten werden mittels Abtastung der rechteckigen 130 oder der Scheibenmultichips 140 sowie der Einzelchips 110 ermittelt und im PC 201 bearbeitet. Die bearbeiteten Daten werden entweder mittels Aufnahme auf den festen Datenträger, z. B., auf der CD 204 oder mittels Übertragung per Internet oder per Lokalnetz auf PC 205 ausgegeben. Aufgrund dieser Daten wird die Datenbank von zahlreichen Diagnosen erstellt. Diese Daten werden z. B. auf Festplatten 206 gespeichert. Diese Information kann per Lokalnetz oder übers Internet durch mehrere Benutzer von ihren PCs 207, z. B. von Laptops aus gleichzeitig benutzt werden.

Zur Erfassung der Diagnosedaten ist es zweckmäßig, solche Scanner einzusetzen, welche mindestens eines der drei bekannten Verfahren realisieren kann, nämlich: Durchlassmessung, Reflexionsmessung und Fluoreszenzmessung.

Die Erkennung des fluoreszenten Signals erfolgt unter Einsatz von einer Erkennungseinrichtung, z. B. einer Versuchsanlage. Die Versuchsanlage besteht aus einem Fluoreszenzmikroskop, einer CCD-Kamera und einem PC. Es kann auch eine Dunkelfeldeinrichtung mit einer angeschlossenen CCD-Kamera [20] sein. Auf diese Anlage wird in der genannten Beschreibung verwiesen. Diese Anlage benutzt das erregende Licht der Laser-LEDs. Die kolorimetrische Erkennung, z. B. mittels einer Biotinhaltigen DNS, erfolgt auch nach dem Auswaschen des Mikrochips unter Einsatz von Abtasteinrichtungen. Das kann, z. B. eine Versuchsanlage sein, die aus einem Scanner, einem PC und einer spezifischen Software besteht. Der PC dient als Mittel zur Auslegung und Speicherung der Diagnoseergebnisse.

Es ist sinngemäß, dass die Ausrüstung die Einrichtungen aus folgender Gruppe enthält: Handscanner zum Strichkode- und Bildeinlesen, automatische Flachbettscanner, Scanner zum Einlesen der Daten von separaten Einzelchips und andere Scanner.

Der Scanner setzt die Bilder aus der Arbeitszone des zum Multichip gehörenden Chip oder der konkreten Cluster aus der Arbeitszone des Chips in die Signale um. Die Signale werden mittels Fluoreszenz-, Durchlass-, Reflexions- und kolorimetrischer Messungen erzeugt. Diese Signale werden nachher in die digitalen Daten umgesetzt. Die Digitaldaten werden auf den PC zur weiteren Bearbeitung gemäß unterschiedlicher Algorithmen übertragen. Die Beschreibung solcher Hard-und Softwaresysteme sind aus der Fachliteratur gut bekannt.

### Auslegung der Ergebnisse

Das Strukturschaubild einer der Ausführungsformen des medizinischen Chips ist in Fig. 6 angeführt. Diese Ausführungsform umfasst den Erfindungsgehalt, ohne ihn jedoch zu beschränken. Die Differenzieroligonukleotide sind in 12 (zwölf) Gruppen je nach der Pathogenart aufgeteilt. Die Signalstärke der gebildeten vollständigen oder unvollständigen Duplexe innerhalb jeder Gruppe ist wesentlich höher als die Signalstärke der negativen Kontrollmittel. Das ermöglicht es, die Identifizierung nach der Artzugehörigkeit der Infektionspathogene sicher durchzuführen. Die Ergebnisdeutung erfolgt aufgrund des Hybridisierungsbildes. Das Hybridisierungsbild wird unter Einsatz einer Versuchsanlage erhalten. Die Anlage beinhaltet z. B. einen Scanner, einen PC und eine spezielle Software. Zur digitalen Bildbearbeitung wird das Programm Medscan-1 verwendet.

Die Ergebnisdeutung erfolgt folgenderweise:
Die DNS-Sondengruppe enthält Oligonukleotide, welche für eines der 12 Infektionsagenzien artspezifisch sind. Die Fluoreszenz- oder kolorimetrischen Signalstärken in jeder DNS-Sondengruppe wird anhand einer speziellen Software mit den Sondengruppen der positiven (max. Signalstärke) und der negativen (min. Signalstärke) Kontrollmittel verglichen. Im Endeffekt wird ein Hybridisierungsbild erhalten, welches für die jeweiligen Gruppen des positiven Kontrollmittels, des negativen Kontrollmittels und der 12 Arten von Infektionsagenzien unterschiedlich ist. Das ermöglicht es, die resultierenden Daten eindeutig auszulegen.
Die Besonderheiten der Hardware- und des Softwaresysteme, welche im Rahmen dieser Erfindung entwickelt wurden, betreffen den Einsatz von Multichips als Gegenstände der Diagnose. Jeder Multichip wird durch eine Menge von Parametern gekennzeichnet. Die Gruppe dieser Parameter umfasst: a) Signalstärkewerte bei jedem konkreten Cluster der Arbeitszone für den jeweiligen Einzelchip, b) Clustermenge in der Arbeitszone jedes Einzelchips und ihre Verteilung über die Forschungsgegenstände, c) Anzahl und Anordnung der positiven und/oder der negativen Kontrollmittel in der Arbeitszone von jedem der Chips, d) Verfahren der Signalstärkedatenerfassung und e) die Abbildung der Chip-Arbeitszone in Form von einer grafischen Darstellung. Diese Parameter werden gemäß bestimmten Algorithmen in einer Datei abgelegt, welche dann in eine Datenbank übertragen wird, z. B. in einem gemeinsamen Rechner einer Klinik. Zusätzlich zu diesen Parametern beinhaltet die Datei den einzigartigen Identifikationskode. Er besteht aus der Kombination des ersten Identifikationskodes jedes Einzelchips und/oder des zweiten und des dritten Identifikationskodes, welche die Multichip-Parameter kennzeichnen.
Die Software identifiziert den einmaligen Multichip-Kode im automatischen oder im Dialog-Modus und bearbeitet die Parameter, welche beim Einscannen des Multichips oder seiner individuellen Bestandteile in die Datei aufgenommen worden sind. Die Bearbeitungsdaten werden in der Datei abgespeichert und, je nach dem bestehenden Datensicherungssystem in jeweiliger Heilanstalt, entweder auf CDs im Archiv (in der Kartei) zusammen mit der Patientenkarteikarte oder in digitaler Form auf den Festplatten des Zentralrechners des Krankenhauses gespeichert. Diese Daten können vom Arzt jederzeit benutzt werden. Dabei erfolgt die Dateisuche durch die Eingabe des Patientenkodes und/oder durch das Einlesen der Strichkode von der Patientenkarteikarte. Nach dem Erhalt der Diagnoseergebnisse können einzelne Chips in der Patientenkarteikarte gespeichert werden. Beim Einlesen des Chip-Strichkodes und bei der Eingabe dieser Daten in den Zentralrechner gibt der Zentralrechner die gesamten Informationen aus der Datei aus, welche alle Parameter und Diagnose-Kode kennzeichnet. Der Arzt kann die Daten, welche in verschiedenen Behandlungsstadien gemäß den Ergebnissen der unabhängigen Diagnosen erhalten worden sind, miteinander vergleichen und die Behandlung bzw. die Medikamenteneinnahme in Übereinstimmung mit den Diagnosedaten korrigieren. Somit ermöglicht der einmalige Kode des Multichips und seinen Bestandteilen, die Software zur Suche nach den erforderlichen Dateien zu vereinfachen und die Diagnosedaten in verschiedenen Formen abzuspeichern, angefangen mit den Computerdateien bis zur Aufbewahrung der Einzelchips während der Behandlung des Kranken.

### Diagnosesatz

Der weitere Schutzgegenstand gemäß dieser Erfindung ist ein Satz zur Identifizierung der Infektionsagenzien je nach ihrer Artzugehörigkeit. Der Satz beinhaltet:
a) mindestens einen individuellen biologischen Mikrochip zur Durchführung einer einmaligen Diagnose oder einen biologischen Multichip zur Durchführung von Parallel- oder Serienselektion, gemäß Punkt 27;
b) Reagenzien, welche aus den Reagenzien zur Probevorbereitung, Reagenzien zur PCR-Durchführung, Reagenzien zur Durchführung der Hybridisierung sowie ihre Kombinationen gewählt werden und
c) spezifische Oligonukleotide als PCR-Primer zur Identifizierung des Infektionsagens je nach der Artzugehörigkeit, mit Sequenzen von SEQ ID NO :1 bis SEQ ID NO: 58.

Der Diagnosesatz enthält zusätzlich eine Einrichtung zur Durchführung einer Hybridisierung und/oder eine Einrichtung zum Scannen und zur Auslegung der Analyseergebnisse. Dabei wird zum Einscannen eine Einrichtung benutzt, welche aus folgender Gruppe gewählt wird: Handscanner, Scanner für individuelle Chips, Flachbettscanner für Multichips, Scanner für Scheiben-Multichips und PC.

### Beispiele

Weiter unten sind Beispiele angeführt, welche den Erfindungsgehalt umfassen, ohne ihn jedoch zu beschränken.

### Beispiel 1:

Anheften der Oligonukleotid-Sonden an die Oberfläche von aminomodifiziertem Glas.

Als Sonden wurden die durch die Phosphatgruppe (p) nach der 5'-Stellung ID SEQ No: 59, 68, 90 und 91 modifizierten Oligonukleotide benutzt. Oligonukleotide wurden in Form von 4-Punkt-Clustern aufgetragen. Die Reaktionsmischung enthält 4 µM von Oligonukleotid und 60 mM von Hydrochlorid 3- Dimethylaminopropyläthylkarbodiimid in 0,1 M 1-Methylimidazol, pH 7,0. Die Reaktionsmischung wurde auf die Glasoberfläche anhand eines Roboters (Xpress Lane, USA) aufgetragen. Das Tropfenvolumen betrug 10 bis 50 nl. Die Glasplatten wurden in einen abgedichteten Behälter gesetzt und bei der Raumtemperatur im Laufe von einer Stunde in einer feuchten Atmosphäre gehalten, um einer Tropfentrocknung vorzubeugen. Danach wurden die Glasplatten im Shaker mit 1 M NaCl-Lösung im Laufe von 10 Minuten abgespült, um die nichtkovalent verbundenen Oligonukleotid-Moleküle zu entfernen. Danach wurden die Glasplatten mit destilliertem Wasser behandelt und bei 4 °C getrocknet.

### Beispiel 2:

Durchführung der asymmetrischen PCR.

Die PCR-Mischung bestand aus 10 mM tris-HCl pH 8,8, 50 mM KCI, 0,8 % Nonidet P40, 2 mM MgCl₂, 100 ng DNS der analysierten Probe, spezifischen Oligonukleotid-Primer ID SEQ No:1, 2, 19, 20, 88 und 89 und 8 Einheiten der Taq- Polymerase in einem Umfang von 20 µl. Die Oligonukleotide ID SEQ No: 1, 19 und 88 wurden in der Konzentration 0,05 µM verwendet. Die Oligonukleotide ID SEQ No: 2, 20 und 89, die mittels Biotin nach der 5'-Stellung modifiziert wurden, wurden in einer Konzentration von 0,25 µM verwendet. Die Reaktion wurde im DNS-Verstärker Mastercycler (Eppendorf, Deutschland) unter den in Tabelle 3 beschriebenen Bedingungen durchgeführt.

**Tabelle 3: PCR-Bedingungen**

| SW-Schritt | Temperatur, ^{°}C | Inkubationsdauer, s | Zykluszahl |
|---|---|---|---|
| 1 | 94 | 180 | 1 |
| 2 | 94 | 20 | 43 |
| 3 | 62 | 20 | |
| 4 | 72 | 30 | |
| 5 | 72 | 180 | 1 |

### Beispiel 3: Hybridisierung.

21 µl der PCR-Mischung wurden mit 3 µl 5 x SSC, 3 µl Formamid, 3 µl 10 % SDS ergänzt. Diese Mischung wurde auf die Arbeitszone des Trägers aufgetragen, mit Objektglas abgedeckt und im Laufe von 1 Stunde bei 45 °C inkubiert. Nach der Beendigung der Hybridisierungsreaktion wurde die Chipoberfläche im Orbitalshaker mit folgenden Lösungen gespült:
A) 0,5 × SSC, 0.1 % SDS - zweimal à 5 Minuten;
B) 5 × SSC, 0.1 % SDS - zweimal à 5 Minuten;
C) 0,1 × SSC, 0.1 % SDS - zweimal à 5 Minuten;
D) 0,1 × SSC - dreimal à 1 Minute.

Nach Abschluss der Spülung wurde der Mikrochip bei Raumtemperatur innerhalb von 5 - 10 Minuten getrocknet.

### Beispiel 4: Durchführung der Peroxydase-Reaktion

Das Ausgangs-Streptavidin-Peroxidase-Konjugat (Imbio, Russland) wurde konsekutiv 200mal mit 1 × SSC mit BSA Gehalt von 1 % verdünnt. Das Konjugat wurde auf die Arbeitszone des Trägers aufgetragen, mit Objektglas abgedeckt und im Laufe von 30 Minuten in der feuchten Atmosphäre bei der Raumtemperatur gehalten. Das ungebundene Konjugat wurde mit 2 x SSC im Laufe von 5 Minuten, mit 1 x SSC im Laufe von 5 Minuten und mit 0,1 x SSC im Laufe von 1 Minute ausgewaschen.

Der Träger wurde danach mit der frisch aufbereiteten Substratlösung Dimethylaminobenzidin übergossen. Dafür wurde 1 mg DAB in 1 ml 0,1 x SSC frühestens 30 Minuten vor der Reaktionsdurchführung aufgelöst. Es wurden auch 20 µl der 3 %-gen Hydrogenperoxid-Lösung zugegeben und durchgemischt. Die Arbeitszone wurde mit der zubereiteten Substratlösung übergossen, mit Objektglas abgedeckt und 10 bis 30 Minuten lang bei Raumtemperatur gehalten. War die Reaktion positiv, dann ließen sich braune Flecken des oxidierten Substrats sehen. Danach wurde das Glas mit destilliertem Wasser abgespült und senkrecht in einen Behälter zum Abtrocknen und zur nachfolgenden Lagerung gesetzt.

### Beispiel 5: Chromogen-Sonde-Erkennung

Um die Abbildungen der Hybridisierungsmikrozonen zu bekommen, wurde der Slide-Scanner Nikon CoolScan 9000ED eingesetzt. Danach wurde die quantitative Bildbearbeitung anhand von Original-SW AnGene vorgenommen. Das Ergebnis der Bildanalyse der Hybridisierungsmikrozone auf einem Glasträger ergibt die gemittelte Farbstärke von 43,9 in den Zonen und die Hintergrundstärke von 1.2 (Bezugseinheiten).

Die Fig. 5 enthält die Darstellung der Hybridisierung einer DNS-Probenmischung. Die DNS-Proben wurden den Abstrichen der Patienten entnommen, welche mit urogenitalen Agenzien angesteckt wurden. Die DNS wurde aus der Mischung ausgesondert. Danach wurde die PCR-Analyse mit einer Mischung aus 13 PrimerPaaren vorgenommen. Die Primer-Paare waren für 12 Pathogene aus folgender Gruppe spezifisch: Chlamydia trachomatis, Neisseria gonorrhoeae, Trichomonas vaginalis, Ureaplasma urealyticum, Candida albicans, Mycoplasma genitalium, Mycoplasma hominis, Gardnerella vaginalis, Streptococcus agalactiae, Human Herpesvirus 1, Human Herpesvirus 2, Human Herpesvirus 4, Human Herpesvirus 5 und DNS des positiven Kontrollmittels. Nach der PCR erfolgte die Hybridisierung auf einem Biochip (sein Strukturschaubild ist in Fig. 6 angeführt). Es ist ersichtlich, dass jeder DNS ihr eigenes individuelles Hybridisierungsbild eigen ist. Es wurde festgestellt, dass die Mischung der DNS-Proben aus den Abstrichen der mit urogenitalen Agenzien infizierten Patienten keine Chlamydia trachomatis, Human Herpesvirus 1, Human Herpesvirus 2 und Human Herpesvirus 4 enthielt. Der dem negativen Kontrollmittel entsprechende Cluster kam nicht zur Geltung.

Die Fig. 6 enthält das Strukturschaubild des medizinischen Chips. Hier ist die Anordnung der Cluster in der Biochip-Arbeitszone abgebildet. Diese Ausführungsform umfasst die gesamte Cluster-Anordnung, ohne sie jedoch darauf zu beschränken.

### Gewerbliche Anwendbarkeit

Das Verfahren zur Identifizierung der Infektionsagenzien auf dem biologischen Mikrochip je nach ihrer Artzugehörigkeit, vorwiegend im urogenitalen Bereich, zeichnet sich gegenüber dem Stand der Technik durch folgende Merkmale vorteilhaft aus:
- schnelle Analyse,
- einfache Methoden zur Vorbereitung der DNS-Probe auf die Hybridisierung,
- die Möglichkeit, gleichzeitig bis zu 15 am meisten verbreiteten Pathogenen zu erkennen,
- die Möglichkeit, die Analyse sowohl im Labor als auch vor Ort (im Freiland) durchzuführen und
- relativ niedrige Kosten.

Die im Text dieser Beschreibung angeführten Texte aus den Patentschriften und Patentanmeldungen gelten als Quellennachweise.

### Quellennachweise

[1] Agatova L. A. usw. Verfahren zur Diagnose der Infektionsschädigung des Körpers. Patentanmeldung RU 2001133477 (2003.08.20).
[2] Yuriev S. Yu. Verfahren der Serologie der aktiven Chlamydia-Infektion bei Schwangeren. Patent RU 2272293 (2006.03.20).
[3] Shipulin G. A. Nährboden für Erkennung von Ureaplasma Urealyticum und Verfahren zur Diagnose vom urogenitalen Ureaplasmose. Patent RU 2265656 (2005.12.10).
[4] Alexandrova N. V. usw. Verfahren zur Diagnose der Infektionen, welche durch das Epstein-Barr Virus verursacht sind. Patent RU 2247390 (2005.02.27).
[5] Koshkin S. V. usw. Verfahren zur Erhöhung der Empfindlichkeit der PCR-Diagnose der urogenitalen Mykoplasmainfektion bei Kranken mit ausgeprägter Exsudativentzündung. Patent RU 2274449 (2006.04.20).
[6] Rothman R.E. usw. Quantitative Analyse zur gleichzeitigen Erkennung und Identifizierung der bakteriellen Infektionen. Patent RU 2004128442 (2005.05.27).
[7] Gharizadeh, B. Target-specific multiple sequencing primer pool for microbial typing and sequencing applications in DNA-sequencing technologies. US Patent Application 20050202436 (September 15, 2005).
[8] Sheiness D. K. et al. Methods for selectively detecting microorganisms associated with vaginal infections in complex biological samples. US Patent 5,700,636 (December 23,1997)
[9] Weisburg W.G. et al. Nucleic acid probes for the detection for genital mycoplasmas. US Patent 5,843,667 (December 1, 1998).
[10] Rossau R. et al. Hybridization probes derived from the spacer region between the 16s and 23s RRNA genes for the detection of non-viral microorganisms. US Patent 6,277,577 (August 21, 2001).
[11] Jannes G, et al. Detection and identification of pseudomonas species using the 16S-23S rRNA spacer. US Patent 6,811,978 (November 2, 2004).
[12] Rothman R.E. et al. Quantitative assay for the simultaneous detection and speciation of bacterial infections. US Patent Application 20030124545 (July 3, 2003).
[13] Bergeron M.G. et al. Species-specific, genus-specific and universal DNA probes and amplification primers to rapidly detect and identify common bacterial and fungal pathogens and associated antibiotic resistance genes from clinical specimens for diagnosis in microbiology laboratories. US Patent Application 20040185478 (September 23, 2004 ).
[14] Schena, M.A. Microarray method of genotyping multiple samples at multiple loci. US Patent Application 20050153318 (July 14, 2005).
[15]. Rava R.P. et al. Methods for concurrently processing multiple biological chip assays. US Patent 6,720,149 (April 13, 2004).
[16] Dellinger D.J. et al. Quality control method for array manufacture. US Patent Application 20050186580 (August 25, 2005).
[17] Remacle J. et al. Detection and/or quantification method of a target molecule by a binding with a capture molecule fixed on the surface of a disc. US Patent Application 20020177144 (November 28, 2002).
[18] Zeleny R. et al. Automatic imaging and analysis of microarray biochips. US Patent 6,215,894 (April 10, 2001).
[19] Cattell H.F. Chemical array fabrication and use. US Patent 6,879,915(April 12, 2005).
[20] Barsky V.E. Fluoreszenzmikroskop. Patent RU 2182328 (2002.05.10).
[21] Beletsky I.P. und andere. Primersatz zur Erkennung und/oder Identifikation der Transgen-Sequenzen der DNS im Pflanzenmaterial und in Pflanzenmaterialhaltigen Produkten (Ausführungsformen), Primer (Ausführungsformen), Primerpaar (Ausführungsformen), Verfahren zur Erkennung und/oder zur Identifikation unter Einsatz dieser Primer (Ausführungsformen) und Einrichtung zur Ausführung des Verfahrens. Patent RU2265668 2005.12.10.
[22] Mirzabekov A.D. usw. Verfahren zur Identifikation der DNS-Transgen-Sequenzen im Pflanzenmaterial und in Produkten auf seiner Basis, Oligonukleotidesatz und Biochip zur Ausführung dieses Verfahrens. Patent RU 2270254 (2006.02.20).
[23] Wolfe, David M. Detection of herpes simplex virus types 1 and 2 by nucleic acid amplification. 20050042601 (February 24, 2005).
[24] Matsumoto T. et al. Method of type-specific detection of herpes simplex virus. 5,354,653 (October 11, 1994).
[25] Groen P. et al. Quantitative epstein barr virus PCR rapid assay. 6,790,952 (September 14, 2004).
[26] Harris R. et al. Detection and quantification of human herpes viruses. PCT Application WO/2002/034953 (02.05.2002).
[27] Smith T. Nucleic acid probes and methods for detecting clinically. PCT Application WO/2000/073499 (07.12.2000).
[28] Farrar G. Genetic suppression and replacement. PCT Application WO/2004/020631 (11.03.2004).
[29] Lindner H. Variants of aminoacylase, nucleic acids coding same, and uses thereof. PCT Appl. WO/2004/113524 (29.12.2004).
[30] Grau O. Oligonucleotide sequences for the specific detection of mollicutes by amplification of preserved genes. PCT Appl. WO/1994/003634 (17.02.1994).
[31] Hirai K. Detection of human cytomegalovirus. JP2000032992 (2000-02-02).
[32] Yoshida. T. Rapid detection of Mycoplasma genitalium, Mycoplasma hominis, Ureaplasma parvum, and Ureaplasma urealyticum organisms in genitourinary samples by PCR-microtiter plate hybridization assay. J. Clin Microbiol. 2003 May; 41 (5): 1850-5.
[33] Purohit A. et al. Method, reagents and kits for the detection of Neisseria gonorrhoeae. US Patent 5,550,040 (August 27, 1996).
[34] Morrison C. et al. Methods and compositions for the detection of Candida spp. US Patent 6,235,890 (May 22, 2001).
[35] Lott T. et al. Nucleic acid probes for detecting Candida species. US Patent 6,242,178 (June 5, 2001).
[36] Hammond P. et al. Nucleic acid hybridization assay probes, helper probes and amplification oligonucleotides targeted to Mycoplasma pneumoniae nucleic acid. US Patent 5,656,427 (August 12, 1997).
[37] Smith T. et al. Detection of herpes simplex virus. US Patent Application 20020164586 (November 7, 2002).
[38] Didkovsky N.A. usw. Herpes-Virus-Infektion: Klinische Bedeutung und Behandlungsgrundsätze. Wissenschaftliches Forschungsinstitut für physikalisch-chemische Medizin, Ministerium für Gesundheitswesen der RF, Moskau, I.M. Sechenovs Moskauer Medizinische Akademie (http://www.medpeterburg.m/news/81231706.html).

## Patentansprüche

**1.** Verfahren zur Identifizierung der urogenitalen Infektion, die folgende Schritte umfasst:
- die Sicherstellung
- einer Probe der zu erforschenden DNS;
- von Hin- und Rückprimer, welche für die Identifikation der Infektionsagenzien im urogenitalen Bereich spezifisch sind, und
- eines biologischen Mikrochips, welcher Differenzieroligonukleotide aufweist,
- die Durchführung von PCR in Anwesenheit der DNS-Probe und der Primer mit Sonden, die für die Identifikation der Infektionsagenzien spezifisch sind,
- die Inkubation des PCR-Produkts mit dem biologischen Mikrochip unter Hybridisierungsbedingungen und
- die Ermittlung der urogenitalen Infektion durch die Erkennung der gebildeten Hybridisierungskomplexe auf dem Mikrochip,
**dadurch gekennzeichnet,**
**dass** der biologische Mikrochip mit Differenzieroligonukleotide mit den Sequenzen SEQ ID NO:59-87 und als spezifische Primer mit Sequenz von SEQ ID NO: 1-58 gewählt werden,
**dass** als PCR ein asymmetrischer PCR benutzt wird,
**dass** der Primer im Hin- und Rück-Primerpaar mit einem Marker versehen wird, der nach der DNS-Hybridisierung erkannt wird,
**dass** die Erkennungsdaten erfasst werden und
**dass** die erfassten Daten zur Identifikation der Infektionsagenzien im urogenitalen Bereich verwendet werden.

**2.** Verfahren nach Anspruch 1,
**dadurch gekennzeichnet,**
**dass** die Probenerkennung in Bezug auf das Vorhandensein von mindestens einer DNS aus dem Gen vorgenommen wird, welcher folgender Gruppe angehört: Chlamydia trachomatis, Neisseria gonorrhoeae, Trichomonas vaginalis, Ureaplasma urealyticum, Ureaplasma parvum, Candida albicans, Mycoplasma genitalium, Mycoplasma hominis, Gardnerella vaginalis, Streptococcus agalactiae, Streptococcus pyogenes, Human Herpesvirus 1, Human Herpesvirus 2, Human Herpesvirus 4, Human Herpesvirus 5.

**3.** Verfahren nach Anspruch 1,
**dadurch gekennzeichnet,**
**dass** die Probe der zu forschenden DNS mindestens aus einer Probe eines menschlichen biologischen Materials ausgesondert wird, wobei das biologische Material Folgendes umfasst: Blut, Biopsie-Material, Speichel, Tränenabsonderungen, Harn, bronchoalveoläre Lavage, Rückenmarkflüssigkeit, Liquor oder ihre Kombinationen.

**4.** Verfahren nach Anspruch 1,
**dadurch gekennzeichnet,**
**dass** der biologische Mikrochip mittels Modifizierung der Mikrochip-Trägeroberfläche erzeugt wird, und dass die Differenzier-Oligonukleotide darauf immobilisiert werden.

**5.** Verfahren nach Anspruch 1,
**dadurch gekennzeichnet,**
**dass** die Oberflächenmodifizierung des Mikrochip-Trägers unter Einsatz einer Lösung von 3-Aminopropyltrietoxysilan vorgenommen wird.

**6.** Verfahren nach Anspruch 1,
**dadurch gekennzeichnet,**
**dass** die Hybridisierung bei einer Temperatur von 35 bis 50 °C in einem Puffer mit 0,5 x bis 5 x SSC; 0,1 % SDS; bis zu 25 % Formamid und/oder bis zu 10 mM EDTA vorgenommen wird.

**7.** Verfahren nach Anspruch 1,
**dadurch gekennzeichnet,**
**dass** der zu ermittelte Marker aus der Gruppe der katalytischen, Ligand-, Fluoreszent- oder radioaktiven Moleküle gewählt wird.

**8.** Verfahren nach Anspruch 7,
**dadurch gekennzeichnet,**
**dass** das katalytische Molekül aus der Gruppe Hämin, Zyankobalamin oder Flavin gewählt wird.

**9.** Verfahren nach Anspruch 7,
**dadurch gekennzeichnet,**
**dass** das Ligand-Molekül aus der Gruppe Biotin, Dioxigenin oder Donitrobenzol gewählt wird.

**10.** Verfahren nach Anspruch 7,
**dadurch gekennzeichnet,**
**dass** das fluoreszente Molekül aus der Gruppe mit FAM, TAMRA, Cy3, Cy5, Cy7, R6G, R110, ROX oder JOE gewählt wird.

**11.** Verfahren nach Anspruch 1,
**dadurch gekennzeichnet,**
**dass** die Erkennung und/oder die Identifikation der Infektionsagenzien mittels einer Vergleichsanalyse der Signalstärke der zu erforschenden Probe, des positiven und des negativen Kontrollmittels vorgenommen wird.

**12.** Spezifisches Oligonukleotid als PCR-Primer zur Identifizierung des Infektionsagens je nach der Artzugehörigkeit im Verfahren zur Identifizierung der urogenitalen Infektion nach den Ansprüchen 1 bis 11 mit den Sequenzen von SEQ ID NO : 1 bis SEQ ID NO: 58.

**13.** Differenzier-Oligonukleotid als Sonde zur Identifizierung des Infektionsagens je nach der Artzugehörigkeit im Verfahren zur Identifizierung der urogenitalen Infektion nach den Ansprüchen 1 bis 11 mit den Sequenzen von SEQ ID NO: 59 bis SEQ ID NO: 87.

**14.** Kombination zur Identifikation des Infektionsagens Chlamydia trachomatis,
**dadurch gekennzeichnet,**
**dass** sie Oligonukleotide mit den Sequenzen von entweder SEQ ID NO: 59 und SEQ ID NO: 1 und SEQ ID NO: 2, oder SEQ ID NO: 60 und SEQ ID NO: 3 und SEQ ID NO: 4 aufweist.

**15.** Kombination zur Identifikation des Infektionsagens Neisseria gonorrhoeae,
**dadurch gekennzeichnet,**
**dass** sie Oligonukleotide mit den Sequenzen von entweder SEQ ID NO: 61 und SEQ ID NO: 5 und SEQ ID NO: 6, oder SEQ ID NO: 62 und SEQ ID NO: 7 und SEQ ID NO: 84, oder SEQ ID NO: 63 und SEQ ID NO: 9 und SEQ ID NO: 10 aufweist.

**16.** Kombination zur Identifikation des Infektionsagens Trichomonas vaginalis,
**dadurch gekennzeichnet,**
**dass** sie Oligonukleotide mit den Sequenzen von entweder SEQ ID NO: 64 und SEQ ID NO: 11 und SEQ ID NO: 12, oder SEQ ID NO: 65 und SEQ ID NO: 13 und SEQ ID NO: 14 aufweist.

**17.** Kombination zur Identifikation des Infektionsagens Ureaplasma urealyticum und/oder Ureaplasma parvum,
**dadurch gekennzeichnet,**
**dass** sie Oligonukleotide mit den Sequenzen von entweder SEQ ID NO: 66 und SEQ ID NO: 15 und SEQ ID NO: 16, oder SEQ ID NO: 67 und SEQ ID NO: 17 und SEQ ID NO: 18 aufweist.

**18.** Kombination zur Identifikation des Infektionsagens Candida albica*n*s,
**dadurch gekennzeichnet,**
**dass** sie Oligonukleotide mit den Sequenzen von entweder SEQ ID NO: 68 und SEQ ID NO: 19 und SEQ ID NO: 20, oder SEQ ID NO: 69 und SEQ ID NO: 21 und SEQ ID NO: 22 aufweist.

**19.** Kombination zur Identifikation des Infektionsagens Mycoplasma genitalium,
**dadurch gekennzeichnet,**
**dass** sie Oligonukleotide mit den Sequenzen von entweder SEQ ID NO: 70 und SEQ ID NO: 23 und SEQ ID NO: 24, oder SEQ ID NO: 71 und SEQ ID NO: 25 und SEQ ID NO: 26, oder SEQ ID NO: 72 und SEQ ID NO: 27 und SEQ ID NO: 28 aufweist.

**20.** Kombination zur Identifikation des Infektionsagens Mycoplasma hominis,
**dadurch gekennzeichnet,**
**dass** sie Oligonukleotide mit den Sequenzen von entweder SEQ ID NO: 73 und SEQ ID NO: 29 und SEQ ID NO: 30, oder SEQ ID NO: 74 und SEQ ID NO: 31 und SEQ ID NO: 32, oder SEQ ID NO: 75 und SEQ ID NO: 33 und SEQ ID NO: 34 aufweist.

**21.** Kombination zur Identifikation des Infektionsagens Gardnerella vaginalis,
**dadurch gekennzeichnet,**
**dass** sie Oligonukleotide mit den Sequenzen von entweder SEQ ID NO: 76 und SEQ ID NO: 35 und SEQ ID NO: 36, oder SEQ ID NO: 77 und SEQ ID NO: 37 und SEQ ID NO: 38, oder SEQ ID NO: 78 und SEQ ID NO: 39 und SEQ ID NO: 40 aufweist.

**22.** Kombination zur Identifikation des Infektionsagens Streptococcus agalactiae,
**dadurch gekennzeichnet,**
**dass** sie Oligonukleotide mit den Sequenzen von SEQ ID NO: 79 und SEQ ID NO: 41 und SEQ ID NO: 42 aufweist.

**23.** Kombination zur Identifikation des Infektionsagens Streptococcus agalactiae und/oder Streptococcus pyogenes,
**dadurch gekennzeichnet,**
**dass** sie Oligonukleotide mit den Sequenzen von SEQ ID NO: 80 und SEQ ID NO: 43 und SEQ ID NO: 44 aufweist.

**24.** Kombination zur Identifikation des Infektionsagens Human Herpesvirus 1 und/oder Human Herpesvirus 2,
**dadurch gekennzeichnet,**
**dass** sie Oligonukleotide mit den Sequenzen von entweder SEQ ID NO: 81 und SEQ ID NO: 45 und SEQ ID NO: 46, oder SEQ ID NO: 82 und SEQ ID NO: 47 und SEQ ID NO: 48 aufweist.

**25.** Kombination zur Identifikation des Infektionsagens Human Herpesvirus 4,
**dadurch gekennzeichnet,**
**dass** sie Oligonukleotide mit den Sequenzen von entweder SEQ ID NO: 83 und SEQ ID NO: 49 und SEQ ID NO: 50, oder SEQ ID NO: 84 und SEQ ID NO: 51 und SEQ ID NO: 52, oder SEQ ID NO: 85 und SEQ ID NO: 53 und SEQ ID NO: 54 aufweist.

**26.** Kombination zur Identifikation des Infektionsagens Human Herpesvirus 5,
**dadurch gekennzeichnet,**
**dass** sie Oligonukleotide mit den Sequenzen von entweder SEQ ID NO: 86 und SEQ ID NO: 55 und SEQ ID NO: 56, oder SEQ ID NO: 87 und SEQ ID NO: 57 und SEQ ID NO: 58 aufweist.

**27.** Biologischer Mikrochip zur Identifizierung der Infektionsagenzien je nach ihrer Artzugehörigkeit, welche folgender Gruppe angehören: Chlamydia trachomatis, Neisseria gonorrhoeae, Trichomonas vaginalis, Ureaplasma urealyticum, Ureaplasma parvum, Candida albicans, Mycoplasma genitalium, Mycoplasma hominis, Gardnerella vaginalis, Streptococcus agalactiae, Streptococcus pyogenes, Human Herpesvirus 1, Human Herpesvirus 2, Human Herpesvirus 4, Human Herpesvirus 5,
**dadurch gekennzeichnet,**
**dass** der biologische Mikrochip einen Träger darstellt, auf dem Differenzieroligonukleotide mit einer oder mehreren der Sequenzen von SEQ ID NO: 59-87 immobilisiert sind.

**28.** Mikrochip nach Anspruch 27,
**dadurch gekennzeichnet,**
**dass** der Chip-Träger aus folgenden Materialien ausgeführt ist: Polymere, Glas, Metalle, Keramik oder ihre Kombinationen.

**29.** Mikrochip nach Anspruch 28,
**dadurch gekennzeichnet,**
**dass** folgende Polymere angewendet sind: Polymethylmethakrylat, Polybutylmethakrylat, Polyvinylchlorid, Polykarbonat, Copolymere von Methylmethakrilat und/oder Copolymere von Butylmethakrylat mit anderen Monomeren, z.B. Styrol, Vinylzyanid.

**30.** Mikrochip nach Anspruch 29,
**dadurch gekennzeichnet,**
**dass** als Polymer Polymethylmethakrylat angewendet ist.

**31.** Mikrochip nach Anspruch 29,
**dadurch gekennzeichnet,**
**dass** als Polymer Polyvinylchlorid angewendet ist.

**32.** Mikrochip nach Anspruch 27,
**dadurch gekennzeichnet,**
**dass** der Chip-Träger in Form von flachen Platten bzw. Folien ausgeführt ist.

**33.** Mikrochip nach Anspruch 27,
**dadurch gekennzeichnet,**
**dass** der Träger als flache Platten bzw. Folien in Form von einem Rechteck, einem Quadrat, einer Scheibe, einem Polygon ausgeführt ist.

**34.** Mikrochip nach Anspruch 33,
**dadurch gekennzeichnet,**
**dass** die Trägerstärke 0,5 bis 5,0 mm beträgt.

**35.** Mikrochip nach Anspruch 27,
**dadurch gekennzeichnet,**
**dass** der Träger als Einzelchip oder Multichip ausgeführt ist.

**36.** Mikrochip nach Anspruch 35,
**dadurch gekennzeichnet,**
**dass** der Träger als ein Multichip mit N vom Träger trennbaren Einzelchips ausgeführt ist.

**37.** Mikrochip nach Anspruch 27,
**dadurch gekennzeichnet,**
**dass** die Differenzieroligonukleotide auf der Trägeroberfläche in Form von Clustern immobilisiert sind.

**38.** Mikrochip nach Anspruch 37,
**dadurch gekennzeichnet,**
**dass** in der Arbeitszone jedes Einzelchips 2 bis 1000 Cluster angeordnet sind.

**39.** Mikrochip nach Anspruch 37,
**dadurch gekennzeichnet,**
**dass** in der Arbeitszone jedes Einzelchips 15 Cluster angeordnet sind.

**40.** Mikrochip nach Anspruch 37,
**dadurch gekennzeichnet,**
**dass** die Zusammensetzung der in der Chip-Arbeitszone angeordneten Cluster aus folgender Gruppe gewählt ist: Sonden-Cluster, Cluster positiver Kontrollmittel und Cluster negativer Kontrollmittel.

**41.** Mikrochip nach Anspruch 40,
**dadurch gekennzeichnet,**
**dass** das Verhältnis zwischen der Anzahl der Cluster mit Sonden und der Anzahl der Cluster mit den darin angeordneten positiven Kontrollmitteln im Bereich 1:1 bis 500:1 gewählt ist.

**42.** Mikrochip nach Anspruch 40,
**dadurch gekennzeichnet,**
**dass** das Verhältnis zwischen der Anzahl der Cluster mit Sonden und der Anzahl der Cluster mit den darin angeordneten negativen Kontrollmitteln im Bereich 1:1 bis 500:1 gewählt ist.

**43.** Mikrochip nach Anspruch 37,
**dadurch gekennzeichnet,**
**dass** die Anzahl der eingeführten Sonden von einem Typ des Differenzier-Oligonukleotides auf der Oberfläche jedes Clusters 1 bis 50 beträgt.

**45.** Mikrochip nach Anspruch 37,
**dadurch gekennzeichnet,**
**dass** die Anzahl der eingeführten Sonden von einem Typ des Differenzier-Oligonukleotides auf der Oberfläche jedes Clusters 4 oder 9 beträgt.

**46.** Mikrochip nach Anspruch 40,
**dadurch gekennzeichnet,**
**dass** das Tropfenvolumen jeder Sonde mindestens 0,005 µl beträgt.

**47.** Mikrochip nach Anspruch 36,
**dadurch gekennzeichnet,**
**dass** die einzelnen Mikrochips mittels Abtrennung des Chips vom Träger gefertigt sind, wobei die Trennung mittels Abbrechens, Abschneidens oder Abhackens erfolgt.

**48.** Diagnosesatz zur Identifizierung der Infektionsagenzien je nach ihrer Artzugehörigkeit, einschließlich:
a) mindestens, eines individuellen biologischen Mikrochips zur Durchführung einer einmaligen Diagnose oder eines biologischen Multichips zur Durchführung von Parallel- oder Serienselektion, welcher Differenzieroligonukleotide mit einer oder mehr Sequenzen SEQ ID NO: 59 bis SEQ ID NO: 87 aufweist,
b) Reagenzien, welche aus den Reagenzien zur Probevorbereitung, Reagenzien zur PCR-Durchführung, Reagenzien zur Durchführung der Hybridisierung sowie ihre Kombinationen gewählt sind,
c) spezifische Oligonukleotide als PCR-Primer zur Identifizierung der Infektionsagenzien je nach ihrer Artzugehörigkeit, welche folgender Gruppe angehören: Chlamydia trachomatis, Neisseria gonorrhoeae, Trichomonas vaginalis, Ureaplasma urealyticum, Ureaplasma parvum, Candida albicans, Mycoplasma genitalium, Mycoplasma hominis, Gardnerella vaginalis, Streptococcus agalactiae, Streptococcus pyogenes, Human Herpesvirus 1, Human Herpesvirus 2, Human Herpesvirus 4, Human Herpesvirus 5, und welche Nukleotid-Sequenzen SEQ ID NO : 1 -: 58 enthalten.

**49.** Diagnosesatz nach Anspruch 48,
**dadurch gekennzeichnet,**
**dass** er zusätzlich eine Einrichtung zur Durchführung der Hybridisierung und/oder eine Einrichtung zum Scannen und zur Auslegung der Analyseergebnisse aufweist, wobei zum Einscannen eine Einrichtung benutzt ist, welche aus folgender Gruppe gewählt ist: Handscanner, Scanner für individuelle Chips, Flachbettscanner für Multichips, Scanner für Scheiben-Multichips und einem PC als Mittel zur Auslegung und Speicherung der Diagnoseergebnisse.
